(19)

Europäisches
Patentamt
European
Patent Office
Office européen
des brevets

(11) **EP 1 819 352 B1**

(12) **EUROPEAN PATENT SPECIFICATION**

(45) Date of publication and mention
of the grant of the patent:
**05.09.2012 Bulletin 2012/36**

(51) Int Cl.:
*A61K 36/58* *(2006.01)*    *A61K 9/00* *(2006.01)*

(21) Application number: **05764528.5**

(22) Date of filing: **21.07.2005**

(86) International application number:
**PCT/BR2005/000132**

(87) International publication number:
**WO 2006/007680 (26.01.2006 Gazette 2006/04)**

(54) **PHARMACEUTICAL COMPOSITIONS FROM CARAPA GUIANENSIS**

PHARMAZEUTISCHE ZUSAMMENSETZUNGEN AUS CARAPA GUIANENSIS

COMPOSITIONS PHARMACEUTIQUES A BASE DE CARAPA GUIANENSIS

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **21.07.2004 BR 0402875**

(43) Date of publication of application:
**22.08.2007 Bulletin 2007/34**

(73) Proprietor: **Fiocruz, Fundação, Oswaldo, Cruz
Manguinhos 21045-900, Rio de Janeiro (BR)**

(72) Inventors:
• **HENRIQUES, Maria das Graças Muller de Oliveira
21045-900, Rio de janeiro, RJ (BR)**
• **MONTEIRO, Carmem Penido
21045-900, Rio de Janeiro, RJ (BR)**
• **SIANI, Antonio Carlos
21045-900, Rio de janeiro, RJ (BR)**
• **GUILHERMINO, Jislaine de Fátima
21045-900, Rio de janeiro, RJ (BR)**
• **RAMOS, Mônica Freiman de Souza
21045-900, Rio de janeiro, RJ (BR)**
• **SAMPAIO, André Luiz Franco
21045-900, Rio de janeiro, RJ (BR)**
• **ROSAS, Elaine Cruz
21045-900, Rio de janeiro, RJ (BR)**
• **DE LIMA, Lucilene Azevedo
21045-900, Rio de janeiro, RJ (BR)**
• **PENNAFORTE, Renato Jabour
35030-390, Governador Valadares, MG (BR)**

(74) Representative: **Fritz, Edmund Lothar
Fritz & Brandenburg
Patentanwälte
Stolbergerstraße 368
50933 Köln (DE)**

(56) References cited:
**EP-A1- 0 872 244        BR-A- 8 605 739
JP-A- 2001 151 634      JP-A- 2003 160 486
US-A- 5 958 421          US-A1- 2002 106 337**

• **HAMMER M L A ET AL: "Tapping an Amazonian
plethora: four medicinal plants of Marajo island,
Para (Brazil)" JOURNAL OF
ETHNOPHARMACOLOGY, ELSEVIER
SCIENTIFIC PUBLISHERS LTD, IE, vol. 40, no. 1,
1 September 1993 (1993-09-01), pages 53-75,
XP023847540 ISSN: 0378-8741 [retrieved on
1993-09-01]**
• **PENIDO C ET AL: "Anti-allergic effects of natural
tetranortriterpenoids isolated from Carapa
guianensis Aublet on allergen-induced vascular
permeability and hyperalgesia" INFLAMMATION
RESEARCH ; OFFICIAL JOURNAL OF: THE
INTERNATIONAL ASSOCIATION OF
INFLAMMATION SOCIETIES THE EUROPEAN
HISTAMINE RESEARCH SOCIETY,
BIRKHÄUSER-VERLAG, BA, vol. 54, no. 7, 1 July
2005 (2005-07-01), pages 295-303, XP019200388
ISSN: 1420-908X**

EP 1 819 352 B1

**Description**

This invention refers to pharmaceutical compositions based on the oil extracted from seeds of *Carapa Guianensis Aublet* and/or based on chemical compounds isolated from this oil, which are responsible for its biological activity, the tetranortriterpenoids. The pharmaceutical compositions of this invention present the following pharmacological activities: anti-allergic, anti-inflammatory, analgesic and immune modulating, and the mentioned compounds substantially reduce the occurrence of side effects and bear a low manufacturing cost.

The pharmaceutical compositions of this invention aim at the treatment, or the prevention, or the inhibition of allergic and inflammatory conditions in human beings, through oral or topic use. In each one of these cases, the compound can be presented in liquid or solid form. And the topic use compound can also be found in the semi-solid form. The compounds for topic use, of this invention, are non-toxic or bear a low toxicity and, which are specially provided in semi-solid form (cream).

The pharmaceutical compositions of this invention constitute an important therapeutic alternative for cutaneous and respiratory allergies, besides acting in different inflammatory reactions of allergic origin, or of infectious origin.

Therefore, these compounds are also used in the symptomatic treatment of rheumatic inflammatory and degenerative processes, several traumas, pain and post-surgery inflammation, acute painful syndromes, among others, and they can be administered orally or in the topic form.

**BACKGROUND OF THE INVENTION**

Approximately 25-30% of the world population presents some kind of allergy, that is, 1.8 billion people around the planet are potential users of anti-allergic medicaments. The most common allergies are respiratory, food and cutaneous.

During the allergic process there is liberation of vasoactive amines, with histamine as a main feature because it is one of the therapeutic target of anti-allergic medicaments. These medicaments are in their majority antihistaminic, that is, antagonistic to receptors H1 of histamine are able to inhibit their effect and prevent symptoms of the allergic response. The development of the first anti-allergenic medicaments occurred in the 60's and nowadays this share of the pharmaceutical market is responsible for the sale of approximately 1.657 million pharmaceutical units of anti-allergic.

The non-steroid anti-inflammatory (AINES) represents the fourth largest market in the Brazilian pharmaceutical industry. The anti-inflammatory existing in the market present several side effects, among which, the most serious are gastric ulceration, hemorrhage and hypersensitivity reactions. Besides that, all of them are developed and registered by the International Pharmaceutical Industry, representing a high cost for the population of developing countries, such as Brazil. Thus, the search for new anti-inflammatory drugs with reduced side effects, besides lower costs is of extreme relevance.

On the other hand, since vegetal species are recognized as sources of compounds with therapeutic activity, they can present an alternative to this case.

In the United States, between 1983 and 1994, 520 new drugs were approved, from which 39% were natural products or products derived from substances extracted from plants. Presently the world market invests 60 billion dollars a year in the manufactures of phytotherapeutic drugs.

World Health Organization (OMS) has also begun developing a program to encourage the use of scientifically validated medicinal plants. Relevant data support the importance of this incentive to traditional medicine, since approximately one third of the population of developing countries do not have access to essential drugs, causing countries such as China, North Korea, South Korea and Vietnam to integrate traditional medicine as an auxiliary in their health system.

Recently, (Brazilian patent application PI0108940 - filed on 23 February 2001) the anti-allergic composition based on plants of the product Aller-7™ has become known (www.InterHealthUSA.com), protected by patent US 6.730.332. It is synergistic anti-allergic compound, based on plants, with extracts of: *Terminalia chebula* fruit (15 to %0% w/w); *Terminalia bellerica* fruit (15 to 50% w/w); *Albizia lebbeck* peel (0.5 to 50% w/w); *Emblica officinalis* fruit (15 to 50%w/w). And it can also contain extracts of: *Piper longum* fruit (0.1 a 5% w/w); Piper *nigrum* fruit (0.1 a 5% w/w); *Zingiber officinale* roots (0.1 a 5% w/w).

According to the document of patent and information on the product Aller-7™ (www.arrowroot.com/aller-7.asp) such plants are known in the Ayurvedic medicine, to treat allergies.

The synergistic composition of the Brazilian patent application PI0108940 aims at, especially, treating rhinitis and asthma. It acts by stabilization of the mastocyte, that is, by prevention of release of histamine, responsible for the allergy manifestation, with the following features:

- intense anti-allergenic activity, which does not only provides relief, especially to allergic rhinitis, allergic asthma and allergic bronchitis, but it also helps to correct subjacent immunologic diseases;

- the control of allergic manifestations such as sneezing, stuffy nose, watery eyes, throat, eyes and nose itching, noisy respiration and breathlessness.
- it does not cause somnolence or immune separation, contrary to other anti-allergenic drugs. It also acts as an anti-inflammatory.

However, the plants composing this synergistic anti-allergenic compound, besides known for treatment of allergies, have their botanical descriptions in the publication Wealth of Asia, and are, therefore, plants of this region. This fact can constitute a restrictive factor when we aim at local production. On the other hand, the synergistic composition of this patent has an antispasmodic activity, but it does not present an analgesic activity.

Considering that Brazil holds 35% of the diversity of plant species of the Earth, it can, therefore, give a decisive contribution to the development of a modern therapeutic for anti-allergenic and anti-inflammatory processes. Besides that, the development of phytotherapeutic drugs or of phytopharmacs presents an essential role in valorizing national raw material with a social and environmental impact.

Therefore, with the aim of overcoming the above-mentioned inconveniences, related to present anti-allergenic and anti-inflammatory drugs, the present invention proposes a new and important therapeutic alternative based on native plants which is as or even more advantageous to those of the state of technique. It is a therapeutic alternative as of phytotherapeutic products of *Carapa guianensis Aublet* for oral or topic use.

*Carapa guianensis* is an Amazonian species of the family of the Meliaceae, commonly known as Andiroba, typical of hydrophile forests, and it can be wild as well as also cultivated. In Brazil, this species occurs as of the state of Tocantins, throughout the Solimões river until the Atlantic coastline, being widely used by its inhabitants, as well as by inhabitants of other South American countries who live in the vicinities of the Amazon forest.

This plant species is commonly used as a repellent, insecticide, antipyretic, vermifuge, against dermatitis, lesions and acne due to its healing and bactericidal properties. Its peels and leaves are used to treat inflammatory reactions such as rheumatism, arthritis and pain, aw well as against infections of the superior respiratory tract such as pneumonia, and also cough and colds. The peel is used to prepare a tea against fever, which also serves as vermifuge. Transformed into powder it treats wounds and it has a healing effect in skin affections, dermatitis, secondary dermal lesions, ulcers, excoriations, acne and it also has antipyretic properties. The following properties are attributed to the leaves: anti-diarrhea, vermifuge, tonic, antipyretic, substitute for quinine in fighting malarial fever, besides being extremely useful against eczema, exanthema and other skin affections (Pio Correa).

It is known that *Carapa guianensis,* or its extracts, associated or not to one or more species of plants, in pharmaceutical compositions for external use, can be applied to:

1) membranes, such as skin, oral cavity, hair, etc. to prevent or to efficiently improve the lack of vigor of the function of these parts of the body, cause by environmental tension or aging (Patent Application JP2001-151634).
2) to hair scalp in order to activate melanocytes of the roots of the hair and stimulate the production of melanin. (Patent Application JP2002-020243).

The seed of andiroba provide a yellowish oil commonly used as repellent and insecticide. For years the Indians used this oil and the vehicle for the application of bixa paint and as hematophagous insect repellent, showing its low toxicity in topic application. In domestic medicine, the oil of *Carapa guianensis* is largely used to be rub on in sore tissues, tumors and muscular lesion. It is characterized by the following therapeutic properties: healing, diuretic, vermifuge, extremely useful against eczema, purgative, anti-rheumatic, against chronic ulcer, against insect bites, tetanus, hepatitis, against skin diseases, it disinfects wounds and acts against swollenness of erysipelas (Pio Correa - *Dicionário das plantas úteis do Brasil).*

The use of an ethno-pharmacological drug of this kind also includes treatment of malaria, leprosy and pneumonia.

The oil of andiroba also is used in cosmetic composition (shampoos, conditioning and moisturizing creams, of the respective patent applications BR PI9301949, BR PI9302004 and BR PI9302006).

The extract of lipid obtained from the core of seeds of andiroba is traditionally used by its anti-inflammatory properties against rheumatic pains and muscular pains, as per patent US 5,958,421, relating to the pharmaceutical or cosmetic compound to be used on the skin and which uses the referred extract to regulate the mechanisms involved in cellulites.

Thus, we observe that until the present moment, andiroba has been used externally, mainly profiting from its anti-inflammatory action.

On the other hand, patent US 4,603,137 describes an isolated substance from plants found in India and from the same family of andiroba, (Meliaceae) with anti-inflammatory, analgesic and immune modulating properties (col. 2, line 49 to 53), and especially immune suppressive. Such alkaloid substance, cromone, is particularly isolated from several parts of the plant *Dysoxylum binectariferum* (for instance: leaves, branches, peel and wood from the trunk, and peel and wood from the roots) and it can be especially used:

- for treatment of patients who present undesirable responses to the immune system, present in the case of autoimmune diseases, which, as a rule, are caused by antibodies and by allergenic or hyper allergenic conditions of the organism, and in the case of chronic inflammatory responses, mainly contributed by macrophages and granulocytes.
- as an immune suppressive agent in transplants of organs or prevention of rejection, with lymphocytes and macrophages playing a important role in this prevention.

Consequently, the respective medicament compounds are also different.

Although the plant bears the same origin of the family of andiroba (Meliaceae), the pharmacological active principle of this patent (alkaloid cromone) is different from those of this invention (oil of andiroba and/or chemical substances isolated from this oil and responsible for its biological activity, the tetranortriterpenoids), because such principles are obtained as of different species. Consequently, the respective medicament compounds are also different.

The compounds of this invention present pharmacological activities: anti-allergenic, anti-inflammatory, analgesic, and immune modulating with reduced side effects. Besides constituting an important therapeutic alternative for skin and respiratory allergies, it also acts both in diverse inflammatory reactions of allergic origin as in inflammatory reactions of infectious origin. Therefore, the compounds of this invention are also used in the symptomatic treatment of rheumatic inflammatory and degenerative processes, several traumas, pain and post surgery inflammations, acute painful syndromes, among others, with the possibility of being orally administered or in the topic form.

**SUMMARY OF THE INVENTION**

This invention aims at providing a pharmaceutical composition characterized by comprising as active ingredients the components (a) or (b) below:

(a) 5 to 30% of an oil extracted from the seeds of *Carapa guianensis Aublet* and 0.01% to 5% of tetranortriterpenoids isolated from the oil of *Carapa guianensis Aublet,* wherein the tetranortriterpenoids are responsible for the pharmaceutical activity;

(b) 0.01% to 5% of tetranortriterpenoids isolated from the oil of *Carapa guianensis Aublet,* wherein the tetranortriterpenoids are responsible for the pharmaceutical activity;

wherein the composition (a) or (b) further comprising as vehicle and/or additives 0.5 to 6% of emulsion basis; 0.2 to 2% of solid vaseline; 0.05 to 0.1% of preserving agents; 2 to 20% of humidifying agents; 0.1 to 10% of 1,8 Cineol; and distilled water qsp 100%;

wherein the dosage form of the pharmaceutical composition (a) or (b) is oral or topic, with anti-allergenic, anti-inflammatory, analgesic and immune modulating activities with substancial reduction of collateral effects and bearing a low manufacturing cost, because they come from a national raw material.

This invention provides this pharmaceutical composition for oral and for topic use and in each one of these cases, the composition can either be in liquid or solid form. And the topic use composition can also be in the semi-solid form.

The pharmaceutical composition of this invention for topic use is atoxic, or of low toxicity, being made by an oil extracted from the seeds of *Carapa guianensis* Aublet and/or of the tetranortriterpenoids, chemical compounds isolated from this oil and responsible for its biological activity, with anti-allergenic, anti-inflammatory, analgesic and immune regulating activities, which are especially provided in the semi-solid form, (cream).

Another concretization of the invention is the use of the composition based on the oil extracted from the seeds of *Carapa Guianensis* Aublet and/or the tetranortriterpenoids, the chemical compounds isolated from this oil are responsible for its biological activity, as an anti-allergenic, anti-inflammatory, analgesic and immune modulating medicament.

Another concretization of this invention is the method of treatment, prevention or inhibition of the allergenic conditions and inflammatory processes comprising the administration of therapeutically effective quantity of the previously referred composition to a human being, needing the referred treatment, prevention or inhibition.

The pharmaceutical compositions of this invention represent, therefore, an important therapeutic alternative for skin and respiratory allergies.

And another important feature of this invention is the fact that it does not act only in different inflammatory reactions of allergic origin, but also in the inflammatory reactions of infectious origin. Therefore, these compositions also are used in the symptomatic treatment of rheumatic, inflammatory and degenerative processes, several traumas, pain and post surgery inflammation, acute pain syndromes, among others, and can be administered orally or in the topic form.

**DESCRIPTION OF FIGURES**

Figure 1 - This figure shows the results of pre-treatment (1 hour, via oral) with oil of *Carapa guianensis* on allergic edema of paw in mice.

Figure 2 - This figure shows the result of pre-treatment (1 hour, via oral) with oil of *Carapa guianensis* on edema of paw induced by histamine in mice.

Figure 3 - The figure in question refers to pre-treatment result (1 hour, via oral) with oil of *Carapa guianensis* on edema of ear induced by histamine in mice.

Figure 4 - This figure shows the pre-treatment results (1 hour, via oral) with oil of *Carapa guianensis* on pleural exudation induced by histamine in mice.

Figure 5 - The figure in question refers to the pre-treatment results (1 hour, via oral) with oil of *Carapa guianensis* on pleural exudation after stimulation with histamine in rats.

Figure 6 - This figure refers to the pre-treatment results (1 hour, via oral) with tetranortriterpenoids isolated from oil of *Carapa guianensis* on edema allergic of paw in mice.

Figure 7 - This figure shows the pre-treatment results (1 hour, via oral) with tetranortriterpenoids isolated from oil of *Carapa guianensis* on cell mobilization in allergic pleurisies in mice.

Figure 8 - This figure shows the pre-treatment results (1 hour, via oral) with tetranortriterpenoids isolated from the oil of *Carapa guianensis* on edema of paw induced by histamine in mice (A) and rats (B).

Figure 9 - The figure in question shows the pre-treatment results (1 hour, via oral) with tetranortriterpenoids isolated from oil of *Carapa guianensis* on edema of ear induced by histamine in mice.

Figure 10 - This figure refers to the pre-treatment results (1 hour, via oral) with tetranortriterpenoids isolated from oil of *Carapa guianensis* on pleural exudation induced by histamine in mice.

Figure 11 - This figure shows the topic pre-treatment results with cream formulations based on oil of *Carapa guianensis* and with tetranortriterpenoids isolated from the same oil on allergic edema of paw in mice (A), and rats (B).

Figure 12 - This figure refers to topic pre-treatment results with creamy formations with oil of *Carapa guianensis* and with tetranortriterpenoids isolated from the same oil on edema of paw induced by histamine in mice (A) and rats (B).

Figure 13 - This figure shows the pre-treatment results (1 hour, via oral) with oil of *Carapa guianensis* on edema of paw induced by carrageenin in mice.

Figure 14 - The figure in question shows the pre-treatment results (1 hour, via oral) with oil of *Carapa guianensis* on edema of paw induced by zimosan in mice.

Figure 15 - This figure refers to the pre-treatment results (1 hour, via oral) with oil of *Carapa guianensis* on pleural exudation and a cell mobilized pleurisies induced by carrageenin in mice.

Figure 16 - This figure shows the pre-treatment results (1 hour, via oral) with tetranortriterpenoids isolated from oil of *Carapa guianensis* on edema of paw induced by the blood platelet activating factor (PAF) in mice.

Figure 17 - The figure in question shows the pre-treatment results (1 hour, via oral) with tetranortriterpenoids isolated from oil of *Carapa guianensis* on edema of paw induced by bradykinin in rats.

Figure 18 - The figure shows the pre-treatment results (1 hour, via oral) with oil of *Carapa guianensis* on hyperalgesia induced by egg albumin in previously sensitized rats.

Figure 19 - This figure shows the pre-treatment results (1 hour, via oral) with oil of *Carapa guianensis* on hyperalgesia induced by histamine in rats.

Figure 20 - This figure shows the pre-treatment results (1 hour, via oral) with oil of *Carapa guianensis* on hyperalgesia induced by carrageenin in rats.

Figure 21 - This figure shows the pre-treatment results (1 hour, via oral) with tetranortriterpenoids isolated from oil of *Carapa guianensis* on hyperalgesia induced by histamine in rats.

Figure 22 - The figure shows the inhibitory effect of treatment with tetranortriterpenoids isolated from *Carapa guianensis* oh the production of nitric oxide by murine macrophages.

Figure 23 - The figure shows an inhibition of production of interferon-γ by murine splenocytes by treatment with tetranortriterpenoids isolated of *Carapa guianensis.*

Figure 24 - The figure shows the effect of treatment with tetranortriterpenoids isolated of *Carapa guianensis* on the production of TNF-α by murine macrophages.

Figure 25 - The figure in question shows the results of treatment with tetranortriterpenoids isolated of *Carapa guianensis* on the proliferation of murine lymphocytes.

Figure 26 - The figure shows the result to inhibitory treatment with tetranortriterpenoids isolated of *Carapa guianensis* on phagocytosis of zimosan by murine macrophages.

Figure 27 - The referred figure shows the effect of the oral administration of tetranortriterpenoids in the gastric mucous of mice C57/B110. The results are expressed in the Mean of Index of Lesions (M.I.L.).

## DETAILED DESCRIPTION OF THE INVENTION

Ethnopharmacological data appoint the use of oil of *Carapa guianensis* for different therapeutic purposes, as described above, including externally, as anti-inflammatory.

However, until the now, the composition provided by this invention had not been described, based on the oil extracted from the seeds of *Carapa guianensis Aublet* and/or tetranortriterpenoids, chemical compounds isolated from this oil and responsible for its biological activity and vehicles and/or pharmaceutically acceptable additives, with anti-allergenic, anti-inflammatory, analgesic and immune modulating activities, with reduced side effects and of low cost, since the derive from national raw material.

These pharmaceutical compositions of this invention aim at the treatment or prevention, or inhibition of the allergic and inflammatory conditions in human beings, through oral or topic use. In each one of these cases the composition can either be in liquid form as solid. And the topic use composition can also be in semi-solid form. The pharmaceutical composition for topic use of this invention is atoxic or of low toxicity.

The anti-allergenic and anti-inflammatory activities of pharmaceutical compositions of this invention are due to its anti-edematogenic activity.

It should be noted that in this invention it is showed the anti-edematogenic activity of the oil extracted from the seeds of *Carapa guianensis Aublet,* as well as from tetranortriterpenoids, when orally administered. Equally, through the topic application, formulations using oil of *Carapa guianensis Aublet* and/or tetranortriterpenoids were also capable to inhibit allergic edema. The compositions for topic use of this invention are atoxic, or present low toxicity, which are especially provided in the semi-solid form. (creamy).

In this invention the anti- allergenic activity of the pharmaceutical compositions based on the oil extracted from the seeds of *Carapa guianensis Aublet* and/or from tetranortriterpenoids, chemical compounds isolated from this oil and responsible for its biological activity, was characterized by the activities of these compounds as antihistaminic, antagonist of bradykinin and antagonist to platelet aggregation factor, mediators involved in the inflammatory response, which was observed in biological essays *in vivo.*

In face of pro-inflammatory stimulus, the pharmaceutical compositions of the invention from the oil of *Carapa guianensis* act inhibiting the cell mobilization, lymphocytes proliferation, phagocytosis and protein overflowing.

The immune modulating activity and also anti-inflammatory, of pharmaceutical compositions of this invention was also characterized by the inhibitory activity of the tetranortriterpenoids on the production of gamma-interferon, of the tumoral necrosis factor (TNF), of nitric oxide, as well as its immune modulation activity was also characterized by the inhibition on the induced proliferation of lymphocytes T, as well as on phagocytosis by murine macrophages. And being evident that this activity is common to the oil extracted from the seeds of *Carapa guianensis Aublet,* from which tetranortriterpenoids are isolated.

Through the inhibitory action of the oil extracted from the seeds of *Carapa guianensis Aublet* and from tetranortriterpenoids an analgesic activity was characterized on hyperalgesia with pharmaceutical compositions of this invention.

Moreover, the compositions of this invention have been proved to present reduction of side effects.

The pharmaceutical compositions of this invention, besides of presenting an important therapeutic alternative to skin allergies (for example: urticaria) and respiratory allergies, act either in the diverse inflammatory responses of allergenic origin, or also in the inflammatory responses of infectious origin. Therefore, these compositions are also used in the symptomatic treatment of rheumatic processes and degenerative, several traumas, pain and post surgery inflammation, acute painful syndromes, among others and it can be orally or topically administered.

The tetranortriterpenoids are known. They are the triterpenes, which have lost four carbon atoms (C-24, C-25, C-26 and C-27), being the carbons C-20, C-21, C-22 and C-23 converted in a furan ring. In this mixture of molecules the following ones are include: 6α-acetoxygedunin, 7-deacetoxy-7-oxogedunin, andirobin, methyl angolensate, gedunin and 6α-acetoxyepoxyazadiradion.

Due to the fact that the tetranortriterpenoids, constituted by the mixture of the referred compounds, present, as previously seen, anti-allergenic, anti-inflammatory, analgesic and immune modulating activities, we can consider that their constituents also present the same characteristics.

For oral administration, the pharmaceutical composition of this invention can be presented as a power, tablets, pills, capsules, or as emulsions, solutions or suspensions. The non-active components in this case include excipients, linking agents, disintegrating agents, diluents, lubricants, etc.

The solid compositions contain the active ingredient in a mixture with non-toxic excipients adequate to the manufacture of tablets, such as amide, lactose, certain kinds of carbonates and bicarbonates, phosphates, talcum, etc. The tablets can be coated or not, depending of the gastro intestinal tract where the disintegration and absorption of the drug may occur.

In case of suspensions, syrups or fluid solutions, excipients such as methyl cellulose, sodium alginate, gum of acacia, lecithin, etc. and one or more additives such as preservatives, coloring, flavoring, thickening, polyols, saccharose, glucose, etc.

The pharmaceutical composition of this invention, for topic use can be in the form of cream, ointment, lotion, gel, solution or suspension. The non-active components are the ones usually used in this case.

This invention is described in detail through the examples presented below. It is necessary to note that the invention is not limited to these examples, but that it also includes variations and modifications within the limits in which it operates.

**Example 1:**

Preparation of Extracts

**(a) Oil of *Carapa guianensis Aublet***

The oil of *Carapa guianensis* utilized of this invention was obtained by mechanical expression of seeds. For use in experiments, aliquots of oil were heated at 40°C until their complete fusion and diluted in a sterile saline solution and Tween 20, at the rate of 1 $\mu$L of tween/mg of the total mass. For the preparation of the treatment solution, the oil needs to be heated. Aiming at guaranteeing the chemical stability of the product, each aliquot of oil was submitted to a heating of 40°C by up to 2 times.

**(b) Tetranortriterpenoids**

The tetranortriterpenoids of the invention can be obtained as of the oil of andiroba or from the bagasse of the seed of andiroba. The processes used in each case are conventional ones.

The oil of andiroba is extracted with acetonitrile, at three stages, minimum, agitate and leave to decant, gathering the supernatant. Filtrate the supernatant and evaporate in rotavapor.

After extraction of the bagasse of the seed of andiroba with pesticide grade hexane by approximately five days, eight hours per day, the material remains in rest for deposit of the solid material. Afterwards a filtration is made and the solid material is placed to dry in a chantry. Then it is strained and left to dry.

The tetranortriterpenoids were made soluble in a sterile saline solution and Tween 20, at the rate of 1 $\mu$L of tween/mg of total mass.

The solutions were prepared for administration in dosages of 12,5; 25; 50; 100 and 200 mg/Kg, in volumes of 200 $\mu$L (for mice) or 400 $\mu$L (for rats).

**Example 2:**

Preparation of solutions, drugs and formulations

The solutions, drugs and formulations used in the experiments made are described as follows.

**(a) Preparation of drugs**

The chloride of promethazine in tablets (Aventis) was macerated, weighed and made soluble in a sterile solution of NaCl 0,9% prepared immediately before use. The cyproheptadine (Sigma) was made soluble in water. Dipyrone was diluted and diclophenate made soluble in fresh water (0,22 $\mu$m). Dexamethasone (Sigma), WEB 2170 (Boehringer-Ingelheim) and HOE 140 (Sigma) were made soluble in a sterile NaCl (0,9%) solution. Promethazine in cream (Rhodia Farma) was directly applied on the paws of the animals.

All drugs were prepared immediately before use.

**(b) Preparation of Solutions**

|  |  |
|---|---|
| **Saline** | |
| NaCl | 0,9 g |
| Distilled water (qsp) pH adjusted to 7,2-7,4 | 100,00 mL |

**Heparanized Saline**

| | |
|---|---|
| Saline | 100,00 mL |
| Heparine pH adjusted to 7,2-7,4 | 2.000 UI |

**Phosphate tampon (PBS)**

| | |
|---|---|
| $NaH_2PO_4.H_2O$ | 0,256 g |
| $Na_2HPO_4.12H_2O$ | 3,004 g |
| NaCl | 8,766 g |
| Distilled water (qsp) pH adjusted to 7,2-7,4 | 1000 mL |

**PBS heparinized**

| | |
|---|---|
| PBS | 1000 mL |
| Heparine pH adjusted to 7,2-7,4 | 20.000 UI |

**PBS / Tween**

| | |
|---|---|
| Tween 20 | 50,0 $\mu$L |
| PBS | 100,0 mL |

**May-Grünwald**

| | |
|---|---|
| May-Grünwald | 0,2 g |
| Methanol | 100,0 mL |

The items above are mixed, heated for 2h at 60°C, and filtered in filtering paper.

**Giemsa**

| | |
|---|---|
| Giemsa | 1,0 g |
| Glycerin | 60,0 mL |
| Methanol | 56,0 mL |

The items above are mixed, heated for 2 h at 60°C, and filtered in filtering paper. For the solution of use, the solution is diluted 10 times.

**Liquid of Türk**

| | |
|---|---|
| Glacial Acetic Acid P.A. | 2,0 mL |
| Violet Crystal (qsp) | 5,0 mg |
| Distilled water (qsp) | 100,0 mL |

**RPMI / gentamicine**

| | |
|---|---|
| RPMI | 10,4 g |
| Gentamicine | 25 mg |
| Deionized water (qsp) | 1,00 L |

**Reagent of Greiss**

| Solution A | Solution B |
|---|---|
| Sulfanilamide - 1,0 g | α-Naftiletilenodiamine 100,00 mg |
| H$_3$PO$_4$ - 5,0 mL Distilled water (qsp) 100,0 mL | Distilled water (qsp) 100 mL |

Solutions A and B are mixed in equal parts (1:1) at the time of use.

**XIX - PBS/Milk**

| PBS/Milk Sigma | 3,0 g |
|---|---|
| Distilled water (qsp) | 100 mL |

**Solution of exposure of ELISA**

| OPD (Dihydrochloride of orto fenilenodiamine) | 5,0 |
|---|---|
| Citrate | 121,5 mg |
| Perborate of sodium | 30 mg |
| Distilled water (qsp) | 10,0 mL |

**Solution of sulphuric acid at 2M**

| H$_2$SO$_4$ P.A. | 166, 67 mL |
|---|---|
| Distilled water (qsp) | 1000,0 mL |

**(a) Preparation of Topic Formulations**

The components and its percentages are described in the formulations. Such preparations can be presented as cream and lotions.

| Oil of *Carapa guianensis* | 5 a 30 % |
|---|---|
| Tetranortriterpenoids | 0 a 5 % |
| emulsion form (alcohols and fatty acids and sodium stearyl lactate) | 0,5 a 6% |
| Solid Vaseline | 0,2 a 2 % |
| Preserving agents | 0,05 a 0,1 % |
| Humidifiers | 2 a 20 % |
| 1,8 Cineol | 0,1 a 10 % |
| Distilled water qsp | 100 % |

The oily components (emulsioned base, humidifiers and preserving agents) were heated at 75°C and the watery components were heated until the temperature of 80°C until their total dissolution. Afterwards, a phase was versed over the other, and homogenized, under agitation (2.000 rpm) until the complete cooling of the formulation.

**Example 3:**

Methodology of assays *in vivo* referring to anti-allergenic activity of oil of *Carapa guianensis* and of tetranortriterpenoids orally administered.

For all procedures *in vivo* described as follows, Swiss male mice were used, weighing from 18 and 25 grams and/or male Wistar rats, weighing between 200 and 300 grams. The animals were furnished by the Central Biotery of *Fundação Oswaldo Cruz* and maintained at the biotery of *Laboratório de Farmacologia Aplicada, Far-Manguinhos,* until the moment of use. The animal had free access to water and animal food and was submitted to alternate cycles of 12h of clear light and darkness, at the temperature of 25°C. The animals were treated with vermifuge (Mebendazol, 20 mg/1000 mL of water) during 3 days, and only used for experimentation after an interval of 3 days. All the experimental procedures were made in accordance with the Ethics of Animal Experiments of Fundação Oswaldo Cruz, RJ.

**a) Test of edema of paw**

The animals were stimulated through interplant injection of stimulus (histamine, bradikinin and PAF - *platelet-activating factor*) in one of the rear paws with volumes of 50 or 100 $\mu$L/paw for mice or rats, respectively. The dosages used were: 100 $\mu$g/paw of histamine, 10 nmol/paw of bradykinin and 1$\mu$g/paw of PAF (we notice that only bradykinin was injected in a volume of 50 $\mu$L). On the counter-lateral side of the paw the same volume of the vehicle (saline sterile free of pyrogen). Thirty minutes or one hour after the stimulus, the edema was analyzed at the digital plethysmograph, through of displacement of fluid (0,5 g NaCl; 3 mL Extran 100%/1L) produced by the insertion of each paw in the measurement tray. Each analysis was performed 3 times.

**b) Test of allergic edema of paw**

This essay was made in animals previously sensitized through an subcutaneous injection (s.c.) in the dorsal region with 200 $\mu$L of a suspension containing 50 $\mu$g of egg albumin + 5 mg (Al(OH)$_3$) diluted in sterile saline free or pyrogens (200 $\mu$L/animal). The edema of paw was induced by an intraplant paw injection of egg albumin (3 $\mu$g/paw, no final volume of 50 $\mu$L) in one of the back paws, fourteen days after sensitization. The counter-lateral paw received an injection with the same volume of the vehicle (solution saline). The edema was analyzes in a digital plethysmograph, through the displacement of fluid (0,5 g NaCl; 3 mL Extran 100%/1L) produced by the insertion of the paw in test in the measurement tray. Each analysis was performed 3 times.

**c) Test of edema of ear**

The ear edema was induced in anesthetized mice (pentobarbital 40 mg/kg, via intravenous, i.v.) with an intravenous injection (i.v.) in the orbital plexus of Evans blue 1% (25 mg/Kg), 24 hours before the stimulus. The animals were stimulated with an intra-dermic injection (i.d.) of histamine (10 $\mu$g/site in 25 $\mu$L) in the superior face of the ear, with glass syringes and needles of diameter 30 ½ G. The opposite ear to the one stimulated received the same volume of vehicle (sterile saline free of pyrogens). Thirty minutes after the stimulus, the animals were sacrificed in the chamber of $CO_2$, and their ears were removed and placed in formamide (500 $\mu$L/ear) for 24 hours for the extraction of Evans blue. The concentration of Evans blue in the supernatant was analyzed through spectrophotometer (SpectraMax®) at $\lambda$ 600 nm. The concentration of Evans blue is determined comparing the optical density é (D.O.) of a pattern curve (from 25,6 to 0,2 $\mu$g/mL).

**d) Pleurisy test**

The pleurisy was induced through intra-thoracic injection (i.t) of histamine (100 $\mu$g/cavity) at the final volume of 100 $\mu$L. The control group received 100 $\mu$L of vehicle (sterile saline). One hour after the stimulus, the animals were sacrificed in a chamber $CO_2$, their thoracic cavities were exposed and washed with phosphate tampon (PBS) heparinized (20 UI/mL). The pleural cavity of the animals was washed with the help of an automatic pipette, with volumes of 1 and 3 mL for mice and rats, respectively. The pleural wash was collected and centrifuged (740 g, 10 minutes) to remove the cells for posterior evaluation of the proteic exudation, as described as follows. In experiences made with mice, they were previously intravenously injected with Evans blue 1% (25 mg/Kg), at the orbital plexus and the proteic exudation was evaluated through overflow of Evans blue, through spectrophotometer at $\lambda$ 600 nm. The concentration of Evans blue was determined comparing the optical density (D.O.) of washes with a pattern curve of Evans blue (of 25,6 a 0,2 $\mu$g/mL). Results were expressed as $\mu$g/mL of Evans blue. For analysis in rats, the exudation of pleural wash was evaluated by the measure of the volume collected from the cavity with the help of a graded syringe and the overflow of proteins was evaluated by the quantification of proteins at the supernatant by the method of Lowry (Lowry et al., 1951). The counting of number of total leucocytes at the pleural wash was made with the help of a Neubauer chamber as of a quota of pleural, diluted 40 times in Türk liquid for lise of erythrocytes. The differential count of mononuclear cells, of neutrophils and of eosinophils was made through colored cell swab by the May-Grünwald-Giemsa method, with help of the optic microscope under objective of immersion (100 x). The results are expressed as number of cells (x 10$^6$) by cavity.

**e) Test of allergic pleurisis**

This essay was performed in previously sensitized animals through a subcutaneous injection(s.c.), at the dorsal region, of 200 $\mu$L of a suspension containing 50 $\mu$g of egg albumin + 5 mg (Al(OH)$_3$) diluted in sterile saline free of

pyrogens (200 μL/animal). Fourteen days after the sensitization the animals were challenged with egg albumin (12,5 μg/cavity) by injection i.t. At a final volume of 100 μL. The control animals received an equal volume of sterile saline. One hour after the challenge with egg albumin, the animals their thoracic cavities exposed and washed with 1 mL (mice) or 3 mL (rats) with PBS heparinized (20 UI/mL). The pleural washed was collected for evaluation of the proteic exudation.

**f) Treatments**

The treatments with oil or tetranortriterpenoids were performed orally (p.o) 1 hour before the stimulation in conscious animals, kept in a previous fasting of 12 hours with free access to water. The treatment solutions were administered with the help of a curve needle with spherical end, with volumes of 200 μL or 400 μL for mice (of 25 g) or rats (of 200 g). The doses used were 200 or 400 mg/Kg for mice and 150 or 300 mg/Kg for rats. The tetranortriterpenoids were administered in doses of 12,5; 25; 50; 100 and 200 mg/Kg. The topic treatment with formulations based on oil of *Carapa guianensis* and/or tetranortriterpenoids was performed with the help of a spatula and the paws treated were immobilized for 5 minutes to allow the absorption of the cream. The topic treatment was made 30 minutes before the stimulation. The following inhibitors were administered orally (p.o.) 1 hour before the stimulus: promethazine (competitive antagonist of receptors H1; 10, 30 or 60 mg/Kg), cyproheptadine (serotoninergic antagonist and of receptors H2; 30 mg/Kg), WEB 2170 (antagonist of PAF; 16 mg/Kg), dipyrone (anti-inflammatory and antipyretic; 100 mg/Kg) and diclophenac of potassium (inhibitor of ciclooxigenase-1 and -2; 100 mg/Kg). A dexamethasone (anti-inflammatory; 2 mg/Kg, p.o.) was administered 24 and 1h before the stimulus, and an administration of HOE 140 (antagonist to bradykinin; 1 μg/paw) was performed immediately before the stimulus, by intraplant injection.

**Example 4:**

Evaluation *in vivo* of the anti-allergenic activity of the oil of *Carapa guianensis* orally administered.

For evaluation of the anti-allergenic activity of the oil of *Carapa guianensis,* the dosages of 200 and 400 mg/Kg were tested for mice and 150 and 300 mg/Kg for rats, depending on the model tested. The solutions of treatment were administered orally (p.o.) 1 hour before the stimulation. For effect of comparison we use antihistaminic chloridrate of promethazine, a competitive antagonist of H1 receptors, as inhibitor of reference. The results were expressed as mean and pattern error of the mean and (AND.P.M.), and statistically analyzed through the analysis of the variance (ANOVA), followed by test of multiple comparisons of Newman-Kewls, or T of Student test with a level of significance minor or equal to 0,05 ($p \leq 0,05$).

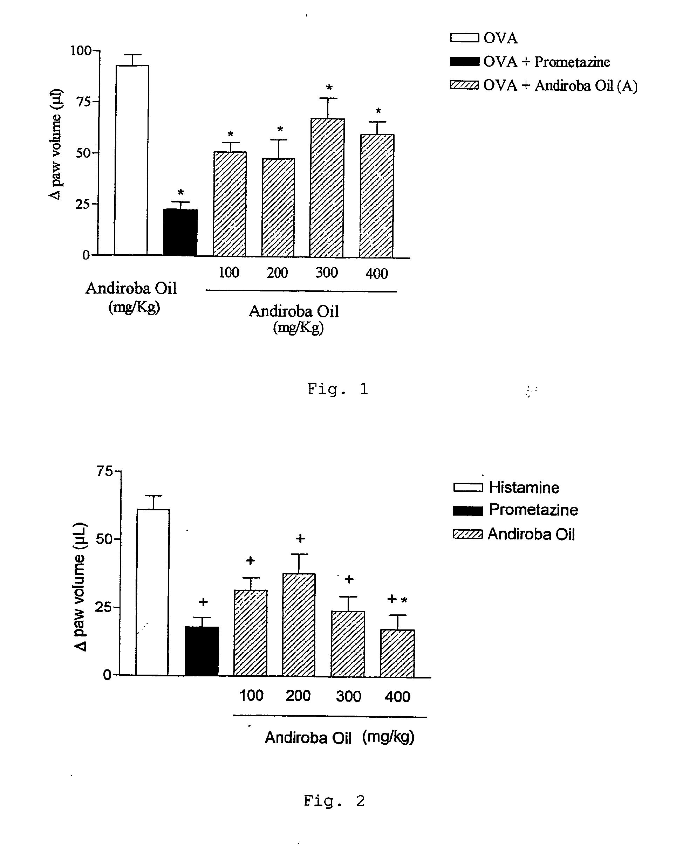

Figure 1 shows the pre-treatment results of mice with oil of *Carapa guianensis,* on doses of 100, 200, 300 and 400 mg/Kg in the model of allergic edema of paw. Each bar shows the mean $\pm$ AND.P.M. of at least 7 animals. The open bar corresponds to the group of animals that received the interplant stimulus with egg albumin. The closed bar corresponds to the group that received oral pre-treatment with promethazine (positive control), and the hachured bars show animals pre-treated with different doses of oil. All groups were sensitized with egg albumin 14 days before of stimulus with egg albumin. Asterisks indicate values statistically different in relation to the positive control group ($p \leq 0,05$), according to the test T Student and Newman Keuls. Figure 1 shows that the interplant injection with egg albumin (3 μg/paw) in mice was able to induce edema of paw, according to reports in literature (Sampaio and col., 1995). This edema was significantly inhibited by oral treatment with promethazine (30 mg/Kg). Pre-treatment with oil of *Carapa guianensis* was able to significantly inhibit the edema induced by egg albumin with doses of 100, 200, 300 and 400 mg/Kg, without showing difference among doses.

**Example 5:**

Evaluation *in vivo* of the antihistaminic activity of oil of *Carapa guianensis* administered orally.

Among mediators responsible for the allergenic response, histamine presents an important role, leading to the increase of vascular permeability, proteic overflow and edema (Bilici et al). Thus, the antihistaminic activity of oil of *Carapa guianensis* was analyzed. Figure 2 shows oral pre-treatment results of oil of *Carapa guianensis* on edema of paw induced by the intraplant stimulation of histamine (100 μg/paw) in mice. Each bar shows the mean $\pm$ AND.P.M. of 8 animals. The closed bar corresponds to the mean of the group stimulated with histamine (positive control). The open bar corresponds to the mean of the group previously treated with promethazine (10 mg/Kg, p.o.), and the hachured bars correspond to groups pre-treated with oil of *Carapa guianensis,* in doses of 100, 200, 300 and 400 mg/Kg. Asterisks indicate those values statistically different with relation to positive control group ($p \leq 0,05$), according to the T Student and Newman Keuls tests. Figure 2 shows that pre-treatment with oil of *Carapa guianensis* was able to significantly inhibit the edema of paw induced by histamine in all doses tested, with the same magnitude of the inhibitor of reference (promethazine).

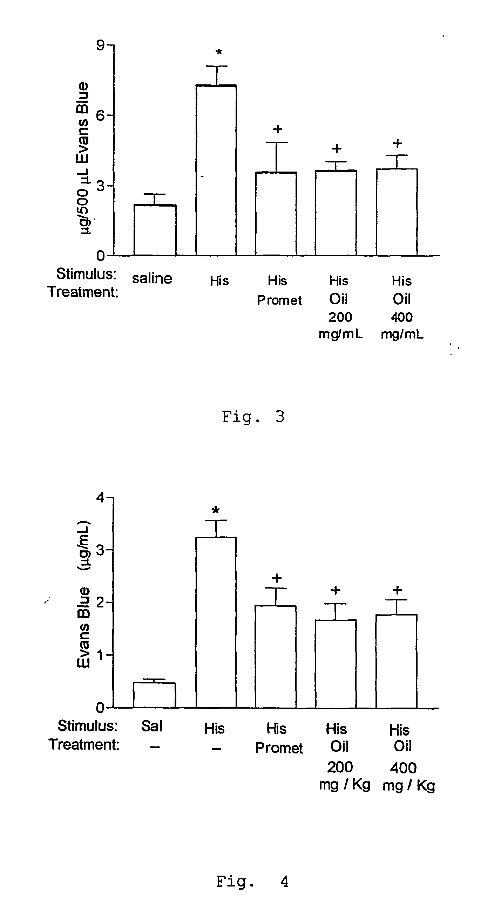

Afterwards the antihistaminic effect of oil of *Carapa guianensis* was evaluated in another experimental model, the ear edema. Figure 3 shows the pre-treatment oral results with oil in doses of 200 and 400 mg/Kg on the ear edema induced by histamine in sensitized animals. Each bar shows the mean ± AND.P.M. of 7 animals. Asterisks indicate values statistically different with relation to the saline group (negative control) and + indicate differences with relation to the group stimulated with histamine (positive control) (p≤0,05), according to the T Student and Newman Keuls tests. Figure 3 shows that the injection of histamine was able to induce the proteic overflow in ears o mice (second column), and that the oral pre-treatment with promethazine (10 mg/Kg, p.o.) was able to significantly inhibit the edema induced by histamine (third column). Equally, pre-treatment with two doses of oil of *Carapa guianensis* (200 and 400 mg/Kg, p. o.) were able to significantly inhibit the edema caused by the injection of histamine in the period of 30 minutes, at the same magnitude as the inhibitor of reference.

Evaluation of anti-histaminic activity of oil of *Carapa guianensis* was also evaluated by the model of pleurisis (da Cunha and col, 2001; Calheiros and col., 2001). Figure 4 shows the pre-treatment results oral with oil with doses of 200 and 400 mg/Kg on proteic exudation in pleurisis induced by histamine in Swiss mice. Each bar shows the mean ± AND.P.M. of 8 animals. Asterisks indicate values statistically different in relation to the group that received injection i.t. of saline (negative control) and + indicate different in relation to the group stimulated with histamine (positive control) (p≤0,05), according to the test of T Student and Newman Keuls. Figure shows that the injection of histamine (100 µg/cavity) was able to induce proteic overflow for pleural activity of mice (second column) significantly different from the negative control group. Oral pre-treatment oral with promethazine (30 mg/Kg, p.o.) (third column) was able to significantly inhibit the proteic overflow induced by histamine. Equally, pre-treatment with two doses of oil of *Carapa guianensis* (200 and 400 mg/Kg, p. o.) were able of significantly inhibit pleural exudation induced by histamine, with the same magnitude as the inhibitor of reference.

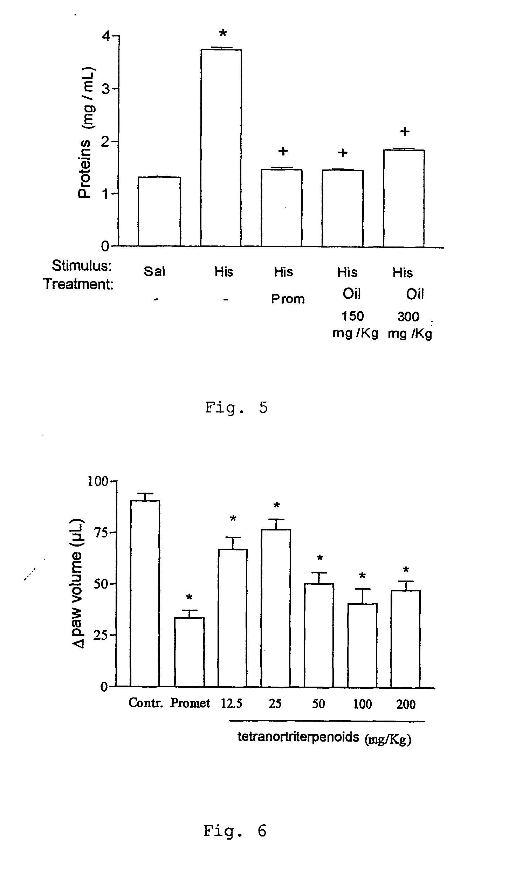

The same experimental model was used in Wistar rats, as showed in figure 5. In the figure in question, each bar corresponds to the mean ± AND.P.M. of at least in 8 animals. The first bar corresponds to the mean of the non-stimulated group (which received saline injection), and the other groups were stimulated with histamine (100 µg/cavity). The second bar corresponds to the animals not treated, the third to the animals treated with the inhibitor of reference (promethazine, 30 mg/Kg) orally. The other bars correspond to the groups that received pre-treatment with oil of *Carapa guianensis* in doses of 200 and 400 mg/kg, orally. The asterisk indicates statistical difference between the group that received intra-thoracic injection of saline (negative control) and + indicate differences in relation to the group stimulated with histamine (positive control) (p≤0,05), according to the test of T Student and Newman Keuls. The figure shows that the injection of histamine was able to induce the proteic overflow of the pleural cavity of rats (second column) significantly different from the negative control group. Oral pre-treatment with the two doses of oil of *Carapa guianensis* (200 and 400 mg/Kg, p. o.) were able to significantly inhibit the proteic overflow induced by histamine, with the same intensity as promethazine (30 mg/Kg, p.o.).

**Example 6:**

Evaluation in vivo of the anti-allergenic activity of tetranortriterpenoids isolated from oil of *Carapa guianensis* administered orally.

The anti-allergenic activity of the tetranortriterpenoids was evaluated through the edema of paw in Swiss mice (figure 6). In figure 6, each bar shows the mean ± AND.P.M. of in at least 8 animals. Results presented demonstrate the edema induced by the injection of egg albumin (3 µg/paw) in sensitized mice (first bar) and the inhibition of edema by oral pre-treatment with promethazine (30 mg/Kg, second bar). The other bars correspond to groups pre-treated orally with tetranortriterpenoids, and demonstrate that doses of 50; 100 and 200 mg/Kg were able to inhibit the allergenic edema, but the doses of 12,5 and 25 mg/Kg did not. Asterisks indicate statistically significant difference (p≤0,05) in relation to the group stimulated and non-treated, according to the test of multiple comparisons Student Newman Keuls.

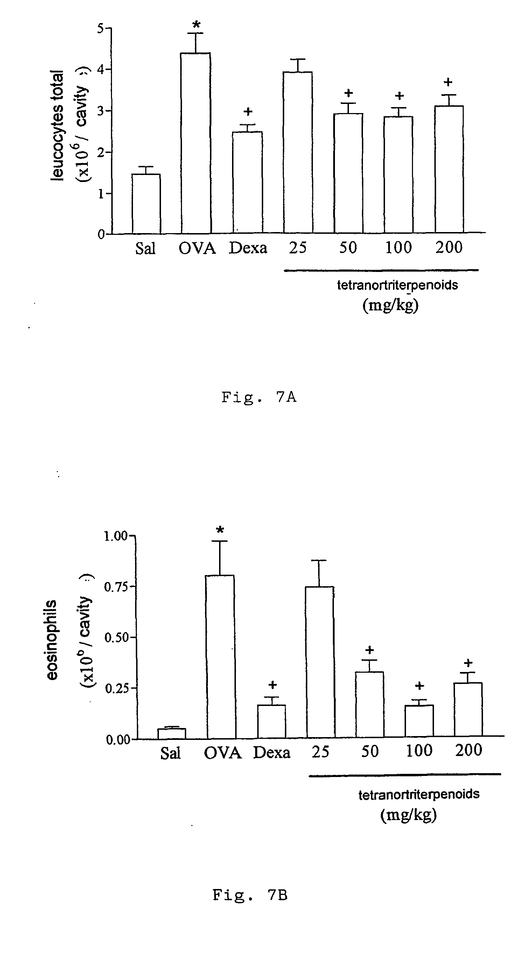

Anti-allergenic activity of tetranortriterpenoids was also evaluated by the allergic pleurisies model (Penido and col., 2001, Sampaio and col., 2000). Figure 7 shows oral pre-treatment results with tetranortriterpenoids with doses of 25, 50, 100 and 200 mg/Kg on pleurisis induced by egg albumin in Swiss mice, during the period of 24 h. Each bar shows the mean ± AND.P.M. of in at least 8 animals. Asterisks indicate values statistically different in relation to the group that received intra-thoracic injection of saline (negative control) and + indicate differences in relation to the group stimulated with egg albumin (positive control) (p≤0,05), according to the test of T Student and Newman Keuls. The figure shows that the injection of egg albumin (12 µg/cavity) was able to induce proteic overflow for the pleural cavity of mice (second column) significantly different from the negative control group. A dexamethasone is anti-inflammatory and the dose of 2 mg/Kg (30 mg/Kg, p.o.) (third column) was able to significantly inhibit the cell accumulation induced by egg albumin. Equally, pre-treatment with the two doses of tetranortriterpenoids (50, 100 and 200 mg/Kg, p. o.) were able of significantly inhibit the accumulation of total leucocytes induced by egg albumin, due to the inhibition of the mobilization of eosinophils for the pleural cavity, at the same magnitude as the inhibitor of reference.

**Example 7:**

Evaluation in vivo of antihistaminic activity of the tetranortriterpenoids isolated from oil of *Carapa guianensis* administered orally.

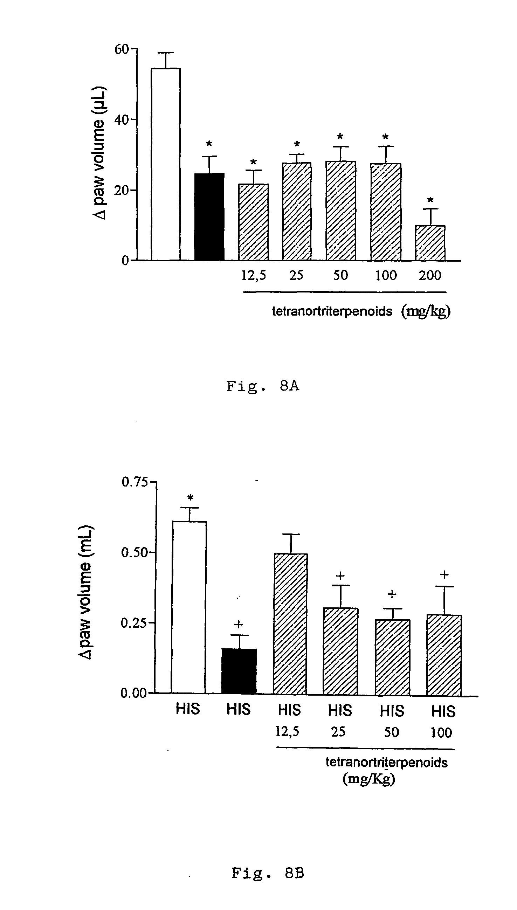

Afterwards the antihistaminic activity of tetranortriterpenoids was evaluated, through models of edema of paw, ear edema and pleurisis. Figure 8 is related to oral pre-treatment results with tetranortriterpenoids on edema of paw induced by histamine in mice (a, 12,5; 25; 50; 100 and 200 mg/Kg) and rats (b, 12,5; 25; 50 and 100 mg/Kg). In the experience with mice, the antihistaminic chlorohydrate of promethazine was used as inhibitor of reference, by means of comparison, as positive control. In the experience with rats, cyproheptadine, an antagonist to the pair of receptors of serotonin ($5-HT_2$) and histamine ($H_1$) was used. Each bar shows the mean $\pm$ AND.P.M. of 8 animals in figure 8a, and of 5 animals in figure 8b. The open bars show the means of animals stimulated with histamine that did not receive treatment (positive control). The closed bars correspond to groups stimulated with histamine, which receive oral pre-treatment with inhibitors of reference (promethazine, 30 mg/Kg for mice and cyproheptadine 30 mg/Kg for rats). The hachured bars show groups of animals stimulated with histamine and pre-treated orally with different doses of tetranortriterpenoids. Asterisks show statistically significant differences ($p \leq 0,05$) in relation to the positive control group. As previously demonstrated, the figure shows that intraplant stimulation with histamine was able to induce the edema in mice (Sampaio and col., 1995) and rats Henriques and col., 1991), and this phenomenon was inhibited by reference compounds used. In mice, all doses of tetranortriterpenoids tested were able to significantly inhibit the edema of paw induced by histamine, while in rats, only doses superior to 12,5 mg/Kg were able to inhibit this reaction. Note: Figure 8

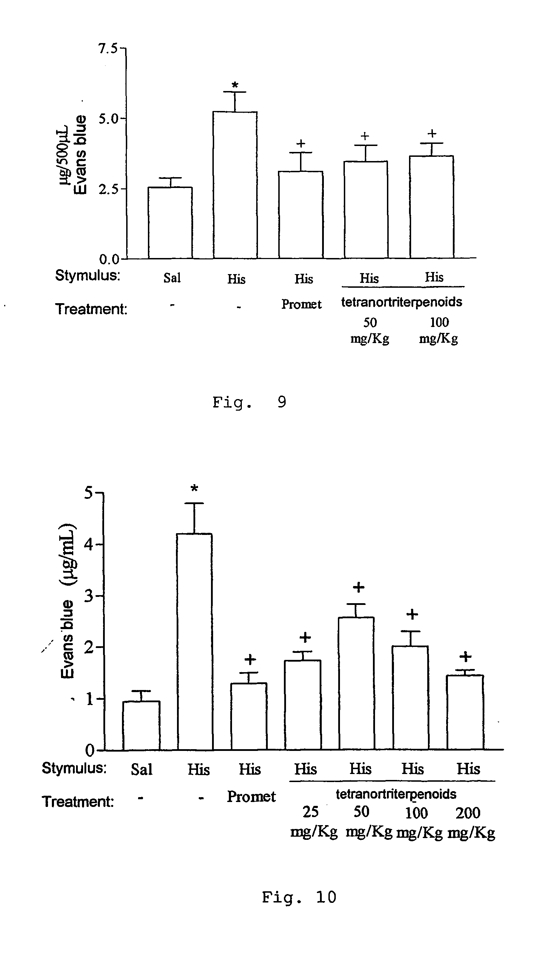

Afterwards, the antihistaminic activity of tetranortriterpenoids was evaluated in ear edema in mice (figure 9). In this figure, each bar corresponds to the mean $\pm$ AND.P.M. of 7 animals. The asterisk indicates the statistical difference ($p \leq 0,05$) between the negative control group (injected with saline) and the positive control (injected with histamine). + indicates the statistical difference in relation to the positive control group. The graph shows that the injection of histamine was able to induce overflow of plasma for ear of mice 30 minutes after the stimulus (second bar), significantly different from the mean of the group injected with saline (first bar). Pre-treatment oral with the two doses of tetranortriterpenoids (50 and 100 mg/Kg) was able to inhibit the formation of edema at the same magnitude as the pre-treatment with the inhibitor of reference (promethazine, 10 mg/Kg, p.o.).

Figure 10 shows the antiedematogenic effect of tetranortriterpenoids in the model of pleurisis induced by histamine in mice. Each bar corresponds to the mean $\pm$ AND.P.M. of 7 animals. The asterisk indicates statistical difference ($p \leq 0,05$) between the negative control group (injected with saline) and the positive control (injected with histamine). + indicates statistical difference in relation to the positive control group. As the figure shows, the intra-thoracic stimulation with histamine (100 $\mu$g/cavity) was able to induce exudation pleural in the period of 1 hour (second bar) when compared with the negative control group (first bar). The other bars correspond to the means of groups orally pre-treated with promethazine (30 mg/Kg, third bar) or with different doses of tetranortriterpenoids (25, 50, 100 and 200 mg/Kg, other bars) and stimulated with histamine one hour after the treatment. We can observe that all doses of tetranortriterpenoids were able to inhibit the plasmatic exudation for the thoracic cavity, without statistical difference among the mean of groups treated.

**Example 8:**

Methodology of essays *in vivo* referring to anti-allergenic activity of creamy formulations of oil of *Carapa guianensis* and of tetranortriterpenoids isolated of oil of *Carapa guianensis* topically administered.

The analysis of the anti-allergenic activity of creamy formulations of oil of *Carapa guianensis* and of tetranortriterpenoids isolated from oil of *Carapa guianensis* for topical use was performed through methodology of allergic edema of paw in Swiss mice and Wistar rats. For this the topic treatment with a particular formulation of this invention was performed in one of the rear paws of the animals. It is important to notice that a control group was included in the study, which received the "blank" (a formulation containing all excipients, not provided with the active principle). The application of cream was made with the help of a spatula, and the rear paws were immobilized for 5 minutes to allow the absorption of the cream. Thirty minutes after the topic treatment, the stimulation was made, through intraplant injection of egg albumin (3 $\mu$g/paw) in previously sensitized animals. The analysis of the edema, was made thirty minutes after the stimulation.

**Example 9:**

Evaluation of *in vivo* essays referring to the anti-allergenic activity of creamy formulations based on oil of *Carapa guianensis* and on tetranortriterpenoids isolated from oil of *Carapa guianensis* topically administered.

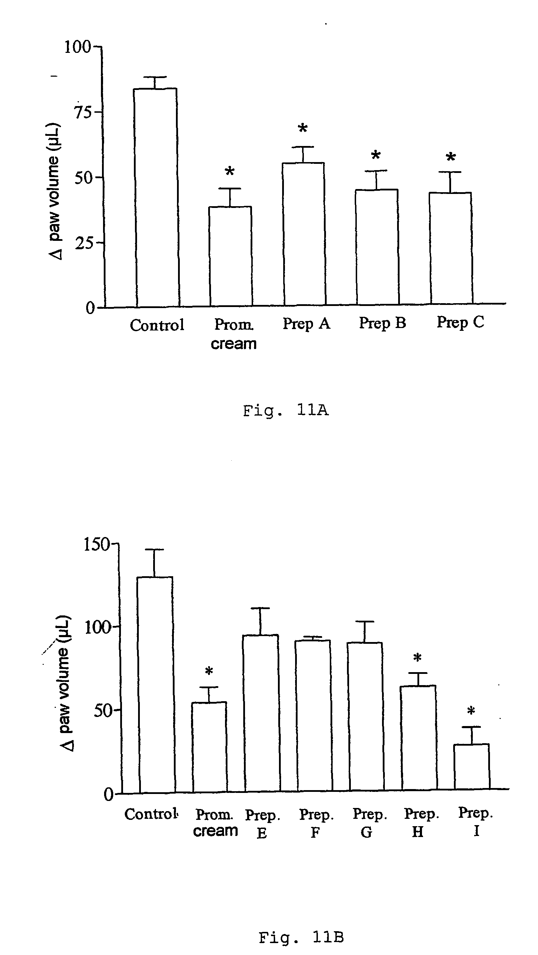

Figure 11 refers to pre-treatment topic results with creamy formulations based on oil of *Carapa guianensis* and on tetranortriterpenoids isolated from oil of *Carapa guianensis* on edema of paw in mice (A) or rats (B) induced by egg albumin ($3\mu$g/paw) in mice. In both experiences, promethazine in cream was used as inhibitor of reference. Each bar shows the mean $\pm$ AND.P.M. of 7 animals by group. The open bar shows the mean of the animals stimulated with egg albumin that did not receive treatment (positive control). It is important to notice that the treatment with the control formulation (excipients) did not induce any alteration in the volume of the paws of the treated animals. The closed bars correspond to groups stimulated with egg albumin, which received topic pre-treatment with the cream of promethazine. The hachured bars show groups of animals stimulated with egg albumin and pre-treated with creamy formulations based on oil of *Carapa guianensis* and on tetranortriterpenoids isolated from oil of *Carapa guianensis.* Asterisks show statistically significant differences ($p \leq 0,05$) in relation to the positive control group. The figure shows that the intraplant stimulation with egg albumin was able to induce the edema in mice, and this phenomenon was inhibited by the cream of promethazine. As it can be observed, creamy formulations based on oil of *Carapa guianensis* and on tetranortriterpenoids isolated from oil of *Carapa guianensis* presented statistically significant anti-allergenic activity when applied in the paw of mice thirty minutes before the stimulation by egg albumin. (Formulations **H** and **I** also showed good results in rats)

**Example 10:**

Methodology of essays *in vivo* referring to antihistaminic activity of creamy formulations based on oil of *Carapa guianensis* and on tetranortriterpenoids isolated from oil of *Carapa guianensis,* topically administered.

The analysis of anti-inflammatory activity of creamy formulations based on oil of *Carapa guianensis* and on tetranortriterpenoids isolated from oil of *Carapa guianensis* for topical use was made through the methodology of edema of paw in Swiss mice and Wistar rats. For this a topic treatment was done in the rear paws of the animals with a test formulation. The formulation containing all the excipients, but not provide to the active principle, which was given to the control group (blank). The application of cream was performed with the help of a spatula, and the rear paws were immobilized for 5 minutes to allow the absorption of the cream. Thirty minutes after the topic treatment, a stimulation was made, through the intraplant injection of histamine (100 $\mu$g/paw). The analysis of edema was performed thirty minutes after the stimulation.

**Example 11:**

Evaluation of essays *in vivo* referring to antihistaminic activity of creamy formulations based on oil of *Carapa guianensis* and on tetranortriterpenoids isolated from oil of *Carapa guianensis,* topically administered.

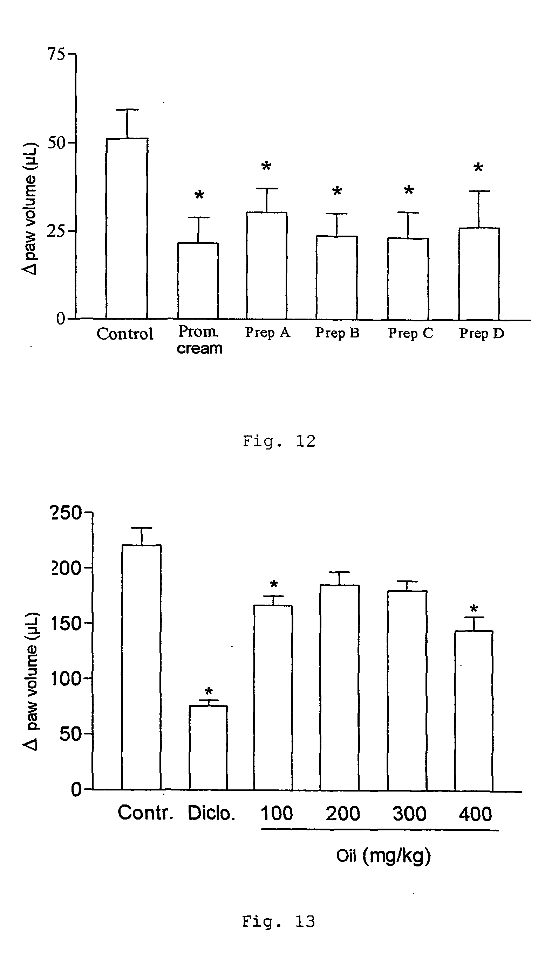

Figure 12 refers to the topic pre-treatment results with creamy formulations based on oil of *Carapa guianensis* and on tetranortriterpenoids isolated from oil of *Carapa guianensis* on edema of paw induced by histamine in mice (A) and rats (B). In both experiences, promethazine in cream was used as inhibitor of reference (positive control). Each bar shows the mean $\pm$ AND.P.M. of 8 animals in figure 12(A), and of 5 animals in figure 12(B). The open bars show the means of the animals stimulated with histamine that did not receive treatment (positive control). It is important to notice that the treatment with control formulation (excipients) did not induce any alteration in the volume of the paws of the treated animals. The closed bars correspond to groups stimulated with histamine, which received topic pre-treatment with cream of promethazine. The hachured bars show groups of animals stimulated with histamine and pre-treated with creamy formulations based on oil of *Carapa guianensis* and on tetranortriterpenoids isolated from oil of *Carapa guianensis.* Asterisks show statistically significant differences ($p \leq 0,05$) in relation to the positive control group. The figure shows that the intraplant stimulation with histamine was able to induce the edema in mice and rats, and this phenomenon was inhibited by the cream of promethazine. As it can be noticed, creamy formulations based on oil of *Carapa guianensis* and on tetranortriterpenoids isolated from oil of *Carapa guianensis* presented significant statistical antihistaminic activity when topically applied on the paw of mice and rats thirty minutes before the stimulation with histamine. [Figure 12 only shows results, for mice (A) the results for rats are absent (B).

**Example 12:**

Methodology of essays *in vivo* referring to anti-inflammatory activity of oil of *Carapa guianensis* orally administered.

The anti-inflammatory activity of oil of *Carapa guianensis* was evaluated through edema of paw and pleurisis, induced by different stimulus, described as follows, in male Swiss mice.

**a) Test of edema of paw**

The animals were stimulated through intraplant injection of stimulus (zimosan or carrageenin) in one of rear paws with the volume of 50 $\mu$L/paw. Doses used were: 500 $\mu$g/paw of zimosan and 300 $\mu$g/paw of carrageenin. In the counter-lateral the same volume of vehicle was injected (sterile saline free of pyrogens). Four hours after the stimulus, the edema was analyzed at the digital plethysmograph, through of displacement of fluid (0,5 g NaCl; 3 mL Extran 100%/ 1L) produced by insertion of each paw in the measurement tray. Each Analysis was performed 3 times.

**b) Test of pleurisis**

Pleurisis was induced through intra-thoracic injection (i.t) of carrageenin (300 $\mu$g/cavity) at the final volume of 100 $\mu$L (Henriques and col., 1991). The control group received 100 $\mu$L of vehicle (sterile saline). Four hours after the stimulus, the animals were sacrificed in of $CO_2$ chamber, their thoracic pleural were exposed and washed with phosphate tampon (PBS) heparinized (20 UI/mL). The pleural cavity of the animals was washed with the help of an automatic pipette, at the volume of 1 mL. The pleural wash was collected and centrifuged (740 g, 10 minutes) to remove cells for posterior evaluation of the proteic exudation, as described as follows. Mice were previously injected intravenously at their orbital plexus with Evans blue 1% (25 mg/Kg), and a proteic exudation was evaluated through of overflowing of Evans blue, through spectrophotometer at $\lambda$ 600 nm. The concentration of Evans blue was determined comparing the optical density (D.O.) of washes with a pattern curve of Evans blue (of 25,6 a 0,2 $\mu$g/mL). Results were expressed as $\mu$g/mL of Evans blue. The count of the number of total leucocytes in the pleural wash was made with the help of a Neubauer chamber as of a quota removed from the pleural wash, diluted 40 times in Türk liquid for the lise of erythrocytes. The differential count of mononuclear cells, of neutrophils and of eosinophils was made through of colored cell swabs through the method of May-Grünwald-Giemsa, with the help of an optical microscope under objective of immersion (100 x). Results are expressed as number of cells (x $10^6$) by cavity.

**Example 13:**

Evaluation of essays *in vivo* referring to the anti-inflammatory activity of oil of *Carapa guianensis* orally administered.

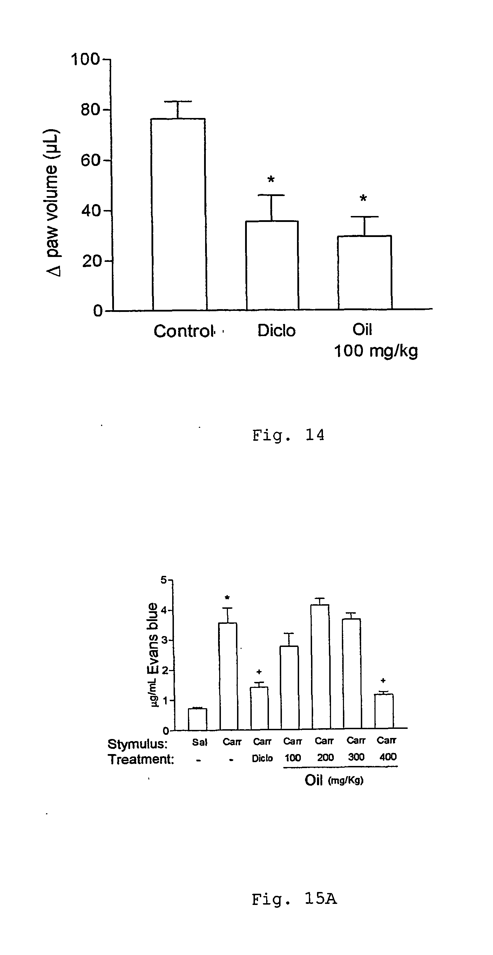

The anti-inflammatory activity of tetranortriterpenoids was evaluated through the edema of paw and pleurisis in Swiss mice. On figures 13 and 14, each bar shows the mean $\pm$ AND.P.M. of 8 animals by group. The open columns show the means of the volumes of paws in the animals of groups stimulated by the injection of carrageenin (300 $\mu$g/paw, fig 13) or zimosan (500 $\mu$g/paw, fig 14) and the closed bars correspond to the inhibition of by oral pre-treatment of edema with diclophenac (100 mg/Kg, second bar). The other bars correspond to groups orally pre-treated with oil of *Carapa guianensis,* and demonstrate that the doses of 100 and 400 mg/Kg were able to inhibit the edema induced by zimosan and a dose of 100 mg/Kg was able to inhibit the edema induced by carrageenin. Asterisks indicate statistically significant differences (p≤0,05) in relation to the group stimulated and non-treated, according to the multiple comparison test of Student Newman Keuls.

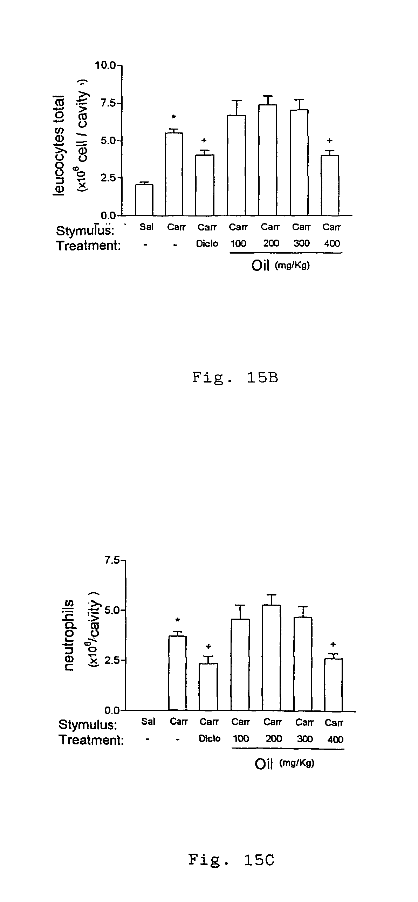

The anti-inflammatory activity of oil of *Carapa guianensis* was also evaluated by the pleurisis model. Figure 15 shows oral pre-treatment results with oil of *Carapa guianensis* at doses of 100, 200, 300 and 400 mg/Kg on pleurisis induced by carrageenin in Swiss mice, in the period of 4 h. Each bar shows the mean $\pm$ AND.P.M. of in at least 7 animals. Asterisks indicate values statistically different in relation to the group that received intra-thoracic injection of saline (negative control) and + indicates differences in relation to the group stimulated with carrageenin (positive control) (p≤0,05), according to the test of T Student and Newman Keuls. The figure shows that the injection of carrageenin (300 $\mu$g/cavity) was able to induce proteic overflow for cavity pleural of mice (second column) significantly different of negative control group. Oral Pre-treatment with diclophenac (100 mg/Kg, p.o.) (third column) was able to significantly inhibit the proteic overflow induced by carrageenin, as the dose of 400 mg/Kg of oil of *Carapa guianensis.* The injection of carrageenin was also able to induce accumulation of leucocytes for the inflammatory focus (B and C, second bar). Pre-treatment with oil of *Carapa guianensis* in the dose of 400 mg/Kg was also able to significantly inhibit the accumulation of total leucocytes induced by carrageenin, due to the inhibition of the mobilization of eosinophils the pleural cavity, at the same magnitude as the inhibitor of reference.

**Example 14:**

Methodology of essays *in vivo* referring to anti-inflammatory activity of the tetranortriterpenoids orally administered.

The anti-inflammatory activity of tetranortriterpenoids isolated of oil of *Carapa guianensis* was evaluated through the edema of paw in Swiss mice and Wistar rats, by different stimuli, described as follows.

**a) Test of edema of paw induced by platelet activation factor**

Swiss mice were stimulated through intraplant injection of 1µg/paw of platelet activation factor (PAF) in one of the rear paws at the volume of 50 µL/paw. In the counter-lateral paw the same volume of vehicle injected (sterile saline free of pyrogens). The stimulus was performed in non-treated animals and animals pre-treated with 16 mg/Kg of antagonist to PAF WEB 2170 (p.o., 100 µL). Thirty minutes after the stimulus, the edema was analyzed by the digital plethysmograph, through the displacement of fluid (0.5 g NaCl; 3 mL Extran 100%/1L) produced by insertion of each paw in the measurement tray. Each analysis was performed 3 times.

**b) Test of edema of paw induced by bradykinin**

Wistar rats were stimulated through intraplant injection of 10 nmol/paw of bradykinin (BK) in one of the rear paws at the volume of 50 µL/paw (Henriques, 1991). In the counter-lateral paw was injected the same volume of vehicle (sterile saline free of pyrogens). The stimulus was performed in non-treated animals and in animals pre-treated through of an intraplant injection of 10 nmol/paw of antagonist to bradykinin HOE 140 at the volume of 50 µL. Thirty minutes after the stimulus, the edema was analyzed on the digital plethysmograph, through the displacement of fluid (0.5 g NaCl; 3 mL Extran 100%/1L) produced by insertion of each paw in the measurement tray. Each analysis was performed 3 times.

**Example 15:**

Evaluation of essays in *vivo* referring to anti-inflammatory activity of tetranortriterpenoids isolated from oil of *Carapa guianensis* orally administered.

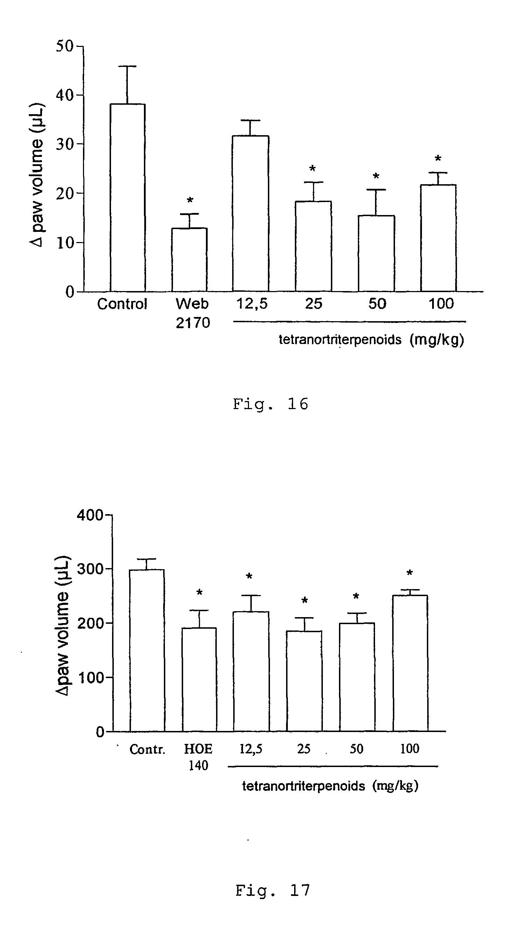

In figure 16, each bar shows the mean $\pm$ E.P.M. of 7 animals. Results presented demonstrate the edema induced by intraplant injection of platelet activation factor (PAF, 1 µg/paw) in Swiss mice (first bar) and the inhibition of edema by oral pre-treatment with the antagonist to PAF, WEB 2170 (16 mg/Kg, second bar). The other bars correspond to groups orally pre-treated with the tetranortriterpenoids, and demonstrate that the doses of 25; 50 and 100 mg/Kg were able to inhibit the edema induced by PAF, but not at the dosage of 12.5 mg/Kg. Asterisks indicate statistically significant differences ($p \leq 0.05$) in relation to the group stimulated and non-treated, according to the test of multiple comparisons Student Newman Keuls.

In figure 17, each bar shows the mean $\pm$ E.P.M. of 7 animals. Results presented demonstrate the edema induced by intraplant injection of bradykinin (10 nmol/paw) in Wistar rats (first bar) and the inhibition of edema by pre-treatment with the antagonist to bradykinin, HOE 140 (10 nmol/paw, intraplant, second bar). The other bars correspond to the groups orally pre-treated with tetranortriterpenoids, and demonstrate that the doses of 12.5; 25; 50 and 100 mg/Kg were able to inhibit the edema induced by bradykinin. Asterisks indicate statistically significant differences ($p \leq 0.05$) in relation to the group stimulated and non-treated, according to the test of multiple comparisons Student Newman Keuls.

**Example 16:**

Methodology of essays *in vivo* referring to the analgesic activity of oil of *Carapa guianensis* orally administered

The anti-allergenic activity was evaluated through an essay of hyperalgesia, performed in a heated plate (Hot Plate, Ugo Basile-Itália model DS-37), with male Wistar rats. The essay is performed through placement of the animals on the hot plate, limited by an acrylic dome of approximately 18 cm in diameter by 40 cm in height.

**a) Hyperalgesia of allergic response**

The induction of the hyperalgesic state was performed through intraplant injection of 10 nmol/paw of egg albumin in animals previously sensitized (12 µg/paw) in one of the rear paws of Wistar rats, at a final volume of 100 µL/paw. In the counter-lateral paw the same volume of vehicle (sterile saline free of pyrogens) was injected. The stimulus was performed in animals non-treated and in animals pre-treated with diclophenac (100 mg/Kg, p.o.). One hour after the stimulus, the animals were placed on a hot plate at 52.5 ± 0.5 °C, and two chronometers are set to record the latency of response of withdrawal of each rear paw. The results were expressed in terms of the variation of latency of withdrawal of right side and left side paws, measured in seconds.

**b) Hyperalgesia induced by histamine**

The induction of a hyperalgesic state was performed through intraplant injection of 100 $\mu$g/paw of histamine in Wistar rats in one of the rear paws, at the final volume of 100 $\mu$L/paw. In the counter-lateral paw the same volume of vehicle (sterile saline free of pyrogens) was injected. The stimulus was performed in animals non-treated and in animals pre-treated with promethazine (30 mg/Kg, p.o.). Thirty minutes after the stimulus, the animals were placed in a hot plate at 52.5 ± 0.5 °C, and two chronometers are set to record the latency of response of withdrawal of each rear paw. Results were expressed in terms of variation of latency of withdrawal of left and right side paws, measured in seconds.

**c) Hyperalgesia induced by carrageenin**

The induction of hyperalgesic state was performed through the intraplant injection of 800 $\mu$g/paw of carrageenin in Wistar rats in one of the rear paws, at the final volume of 100 $\mu$L/paw. In the counter-lateral paw the same volume of vehicle (sterile saline free of pyrogens) was injected. The stimulus was performed in animals non-treated and in animals pre-treated with dipyrone (100 mg/Kg, p.o.). Three hours after the stimulus, the animals were placed in a hot plate at 52.5 ± 0.5 °C, and two chronometers are set to record the latency of response of withdrawal of each rear paw. Results were expressed in terms of variation of latency of withdrawal of left and right side paws, measured in seconds.

**Example 17:**

Evaluation of essays *in vivo* referring to analgesic activity of oil of *Carapa guianensis* orally administered

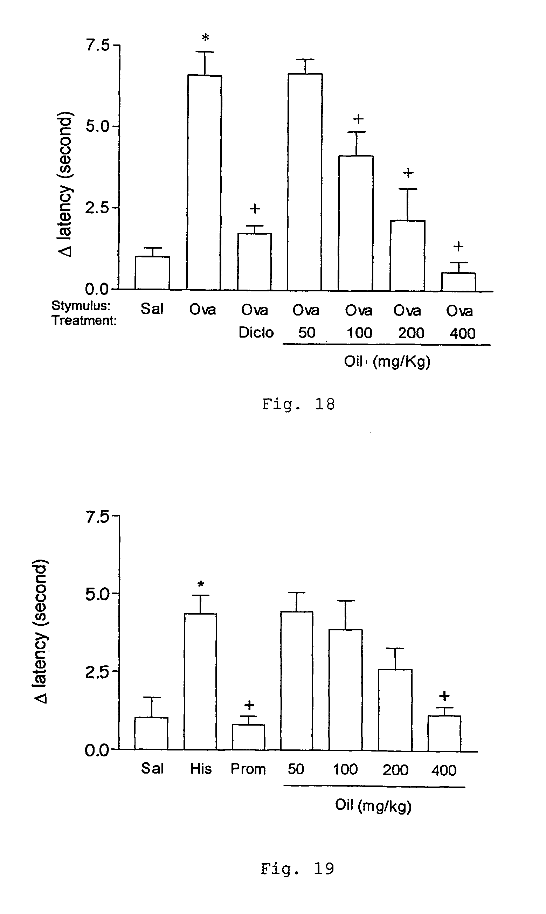

In figure 18, each bar shows the mean ± E.P.M. of 7 animals. Results presented demonstrate hyperalgesia induced by intraplant injection of egg albumin (12 $\mu$g/paw) in Wistar rats (second bar) and the inhibition of hyperalgesia by pre-treatment with diclophenac (100 mg/Kg, p.o., third bar). The other bars correspond to groups orally pre-treated with oil of *Carapa guianensis,* and demonstrate that the doses of 100, 200 and 400 mg/Kg were able to inhibit the hyperalgesic state induced by egg albumin. The asterisk indicates statistically significant differences ($p{\leq}0.05$) in relation to the group non-stimulated, and + shows a statistical difference between the group stimulated and non-treated, according to the test of multiple comparisons Student Newman Keuls.
Figure 19 shows hyperalgesia induced by intraplant injection of histamine (100 $\mu$g/paw) in Wistar rats. Each bar shows the mean ± E.P.M. of 7 animals. A third bar shows the inhibition of hyperalgesia by pre-treatment with promethazine (30 mg/Kg, p.o.). The other bars correspond to the groups orally pre-treated with oil of *Carapa guianensis* (with doses of 50, 100, 200 and 400 mg/Kg). Only the dosage of 400 mg/Kg was able to inhibit the hyperalgesic state induced by histamine, at the same intensity as the inhibitor of reference. The asterisk indicates statistically significant differences ($p{\leq}0.05$) in relation to the non-stimulated group, and + shows the statistical difference between the group stimulated and non-treated, according to the test of multiple comparisons Student Newman Keuls.

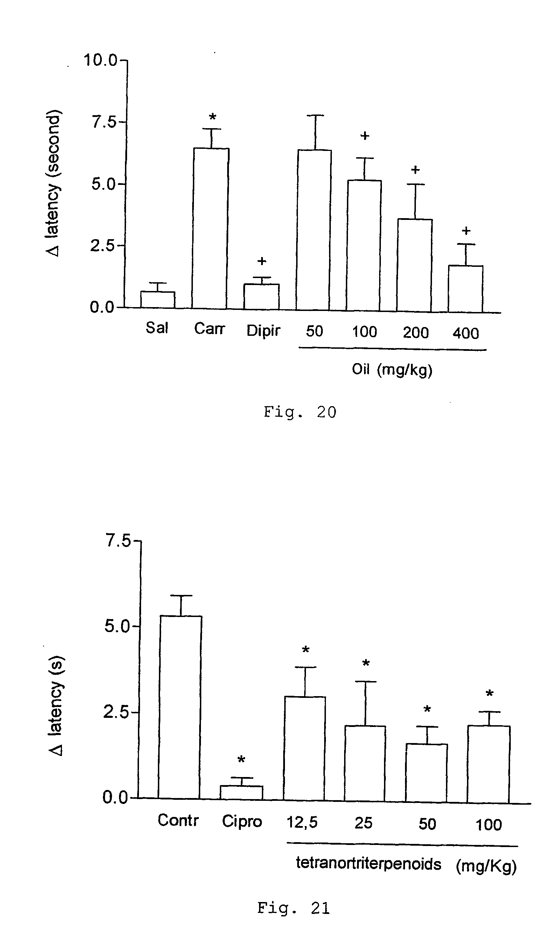

In figure 20, each bar shows the mean ± E.P.M. of 7 animals. Results presented demonstrate hyperalgesia induced by intraplant injection of carrageenin (800 $\mu$g/paw) in Wistar rats (second bar) and the inhibition of hyperalgesia by pre-treatment with dipyrone (100 mg/Kg, p.o., third bar). The other bars correspond to the groups orally pre-treated with oil of *Carapa guianensis* (with doses of 50, 100, 200 and 400 mg/Kg). Doses of 100, 200 and 400 mg/Kg were able to inhibit the hyperalgesic state induced by carrageenin. The asterisk indicates statistically significant differences ($p{\leq}0.05$) in relation to the group non-stimulated, and + shows a statistical difference between the group stimulated and non-treated, according to the test of multiple comparisons Student Newman Keuls.

**Example 18:**

Methodology of essays *in vivo* referring to analgesic activity of tetranortriterpenoids isolated from oil of *Carapa guianensis,* orally administered.

**a) Hyperalgesia induced by histamine**

The induction of a hyperalgesic state was performed through intraplant injection of 100 $\mu$g/paw of histamine in Wistar rats in one of the rear paws, at a final volume of 100 $\mu$L/paw. In the counter-lateral paw the same volume of vehicle (sterile saline free of pyrogens) was injected. The stimulus was performed in animals non-treated and in animals pre-treated with cyproheptadine (30 mg/Kg, p.o.). Thirty minutes after the stimulus, the animals were placed in a hot plate at 52.5 $\pm$ 0.5 °C, and two chronometers are set to record the latency of response of withdrawal of each rear paw. Results were expressed in terms of variation of latency of withdrawal of left and right side paws, measured in seconds.

**Example 19:**

Evaluation of essays *in vivo* referring to analgesic activity of tetranortriterpenoids isolated from oil of *Carapa guianensis* orally administered.

In figure 21, each bar shows the mean $\pm$ E.P.M. of 7 animals. The first bar in the figure shows the induction of hyperalgesic state, and a second bar its inhibition by pre-treatment with the inhibitor of reference (cyproheptadine, 30 mg/Kg, p.o.). The other bars demonstrate that pre-treatment with tetranortriterpenoids with doses of 12.5; 25; 50 and 100 mg/Kg was able to inhibit hyperalgesia induced by histamine.

**Example 20:**

Methodology of essays *in vitro* referring to anti-inflammatory immune modulating activity of tetranortriterpenoids isolated from oil of *Carapa guianensis*

**a) Essay of Production of NO**

Mice Balb/c received an intraperitoneal injection of thioglycolate at 3% (1mL) and 72h after peritoneal macrophages collected through wash with 5mL of RPMI1640 sterile. Cells obtained from the peritoneum were plated (2.5 x $10^5$ cells/well) in a plate of 96 wells and incubated for one hour in an oven of $CO_2$. After this period, non-adherent cells were collected by wash and the adherent cells were stimulated with LPS (37.5ng/mL) in an environment rich in interferon and/or with tetranortriterpenoids (1, 10, 100$\mu$g/mL) and incubated for 24 hours in an oven of $CO_2$. Part of supernatant free of cells was collected after centrifugation (2 min; 2800rpm), transferred (100$\mu$L) to another plate where Greiss reagent was added (100$\mu$L). Determination of concentration of nitrite was performed in a micro plate reader at 540nm, through comparison with a pattern curve of sodium nitrite. Data were analyzed through analysis of variance ANOVA, student-Neuman-Keuls or test of T-student. Results were expressed as mean $\pm$ pattern error of the mean (EPM). Values of p $\leq$ 0.05 were considered significant.

**b) Production of Interferon-$\gamma$ by splenocytes**

Spleens of male Balb-c mice were removed in a sterile environment in a Petri plate containing 3mL of environment RPMI1640 with gentamicine (25$\mu$g/mL). In the laminar flux, the spleens were individually dissociated. Cell suspensions were gathered in a pool in sterile tubes of 15mL. The suspension was left to sediment for 5 minutes and after this period the supernatant was collected. The cell suspension was centrifuged for 10 minutes at 400g; and the pellet of cells was re-suspended with 4 mL of RPMI1640/gentamicine. In another tube, 2mL of histopaque 1077 was placed and then a cell suspension. After centrifugation for 30 minutes at 400g, the cells at the interface between the mean of culture and the histopaque 1077 were collected, washed (10 minutes at 1500rpm) and re-suspended in 1mL of RPMI 1640 supplemented for counting of cell viability by Trypan blue method.
After obtaining and counting cell viability, the cells were seeded in plates of 96 wells (2.5 x $10^5$/well), incubated for 30 minutes and the Con A (0,4$\mu$g/well) was added in the presence or not of the sample test (1, 10, 100$\mu$g/mL). For determination of the production of IFN-$\gamma$, the cells were maintained in culture for 24 hours after the stimulation, the plate was centrifuged (2 min; 2800rpm) and the supernatant, free of cells was collected for dosage of IFN-$\gamma$ by ELISA.

**c) Essay of Production of TNF-$\alpha$**

Balb/c mice received intraperitoneal injection of thioglycolate at 3% (1mL) and 72h after peritoneal macrophages were collected through wash with 5mL of sterile RPMI1640. Cells obtained from the peritoneum were plated (2.5 x $10^5$ cells/well) in a plate of 96 wells and incubated for one hour in a stove of $CO_2$. After this period, the non-adherent cells were collected by wash and the adherent cells were stimulated with LPS (37.5ng/mL) and/or tetranortriterpenoids (1, 10, 100 $\mu$g/mL) and incubated by 24 hours in a stove of $CO_2$. After this period, the plate was centrifuged (2 min; 2800rpm) and part of the supernatant free of cells was collected (100 $\mu$L) and quoted for dosage of TNF-$\alpha$ by the method of ELISA.

**d) Dosage of TNF-$\alpha$ or IFN-$\gamma$ by ELISA of Capture**

For dosage of citokynes, 4$\mu$g/mL of antibody monoclonal anti- TNF-$\alpha$, or IFN-$\gamma$ purified was diluted in a Na$_2$HPO$_4$ (0.1M; pH 9.2) tampon, distributed in a plate of 96 wells (50$\mu$L/well; Maxisorp NUNC) and incubated at 4°C for 18h. After this period the plate was washed in PBS Tween (0.05%) and incubated for 1h at room temperature with a solution of skimmed milk PBS 3% (100$\mu$L/well). After a new wash with PBS Tween (0.05%; PBS-T), the plate was incubated with supernatants duplicated (100$\mu$L) and incubated at 4°C for 18h. After 24 hours, the plate was washed with PBS-T and incubated for 1 hour at room temperature with 100$\mu$L of antibody monoclonal biotinylated anti- TNF-$\alpha$ or IFN-$\gamma$ (0.4$\mu$g/mL). The plate was then washed with PBS-T and incubated with 50 $\mu$L of streptoavidine peroxidase (dilution 1: 800) for 30 min in room temperature. The exposure was performed by the addition of 100$\mu$L of citrate/perborate tampon of sodium containing OPD (0.5mg/mL). The blocking of the response was performed by the addition of 100 $\mu$L of H$_2$SO$_4$ 2M, and reading was performed in a spectrophotometer at 490 nm. The concentration of cytokines at the supernatant was determined from comparison with a pattern curve TNF-$\alpha$ or IFN -$\gamma$ recombinants, through Soft Max Pro program of analysis. Data was analyzed through analysis of variance ANOVA, student-Neuman-Keuls or test of T-student. Results were expressed as mean $\pm$ pattern error from the mean (EPM). Values of $p \leq 0.05$ were considered significant.

**e) Test of proliferation of lymphocytes**

Splenocytes were obtained as described in item b of this example. $2.5 \times 10^5$ cells were seeded by well in a plate of 96 wells. The plate was incubated by 30 minutes in a stove at 37°C and 5%CO$_2$ was added as sample tests (0.1 - 100$\mu$g/mL) in the presence or not of Concanavaline A (With A, 0.4$\mu$g/well). The cells were maintained in the stove and after 72 hours, a 1$\mu$Ci/well of tritiated thymidine was added. After 18h of the addition of thymidine, the cells were transferred for a membrane, with help of Cell Harvester (Packard). The radioactivity reading was performed by scintillation (TopCount NXT; Packard) and data was expressed as counting per minute (CPM). Data was analyzed through analysis of variance ANOVA, student-Neuman-Keuls or test of T-student. Results were expressed as mean $\pm$ pattern error from the mean (EPM). Values of $p \leq 0.05$ were considered significant.

**f) Essay of Phagocytosis**

For the analysis of tax of phagocytosis of macrophages, plates of 24 wells were used, containing, in each well, a glass lamina with 13mm diameter. For use of laminas, they were previously washed in 0.1% solution of Extran Neuter (Merck), and heated for approximately 35 minutes. The operation was duplicated with distilled water. The laminas were dried in a stove and immersed for 18 hours in a solution of nitric acid at 0.1%. After this treatment, they were rinsed with distilled water, dried in a stove and irradiated (2500 rad). $2 \times 10^5$ cells/well were seeded in the presence of IFN-$\gamma$ (10 Unit/mL), tetranortriterpenoids (100$\mu$g/mL) or as control, mean RPMI 1640 supplemented. After 1 hour in the stove at 37°C (5% CO$_2$), 50$\mu$l of zimosan was added (final concentration of $10^6$ particles/mL). This suspension of zimosan was made at 2.5mg/mL in sterile PBS, centrifuged for 15 minutes (3500 rpm), the pellet was re-suspended in 1mL of sterile PBS. After being taken to the ultrasound (10 minutes), a quota of the suspension was removed and diluted to count the number of particles free of zimosan in Neubauer chamber under optical microscopy (objective of 20 times). After the addition of zimosan, the culture was once again taken to the stove for 1 more hour. After this period the laminas were processed for evaluation of phagocytosis. For evaluation of laminas, the cells were washed with PBS and fixed with paraformaldehyde 2% for 30 minutes. After a new wash, the cells were colored with Hematoxiline-Eosine. After coloring, the laminas were washed in fresh water, placed on a microscope lamina and taken to the microscope for cell counting.
In order to quantify the rate of phagocytosis the number of particles found in 200 cells was counted. Cells presenting, in their interior, a number equal or greater than 4 particles of zimosan were accepted as positive for phagocytosis. Results were expressed as percentage of phagocytosis of the control group as the formula below:

$$\% \text{ phagocytosis} = \frac{\text{n. of positive cells in treated group}}{\text{n. of positive cells in environment group}} \times 100$$

Data was analyzed through the analysis of variance ANOVA, student-Neuman-Keuls or test of T-student. Results were expressed as mean $\pm$ pattern error from the mean (EPM). Values of $p \leq 0.05$ were considered significant.

**Example 21:**

Evaluation of essays *in vitro* referring to the anti-inflammatory and immune modulating activity of tetranortriterpenoids isolated from oil of *Carapa guianensis*

**a) Evaluation of influence of tetranortriterpenoids isolated from oil of *Carapa guianensis* on the production of nitric oxide**

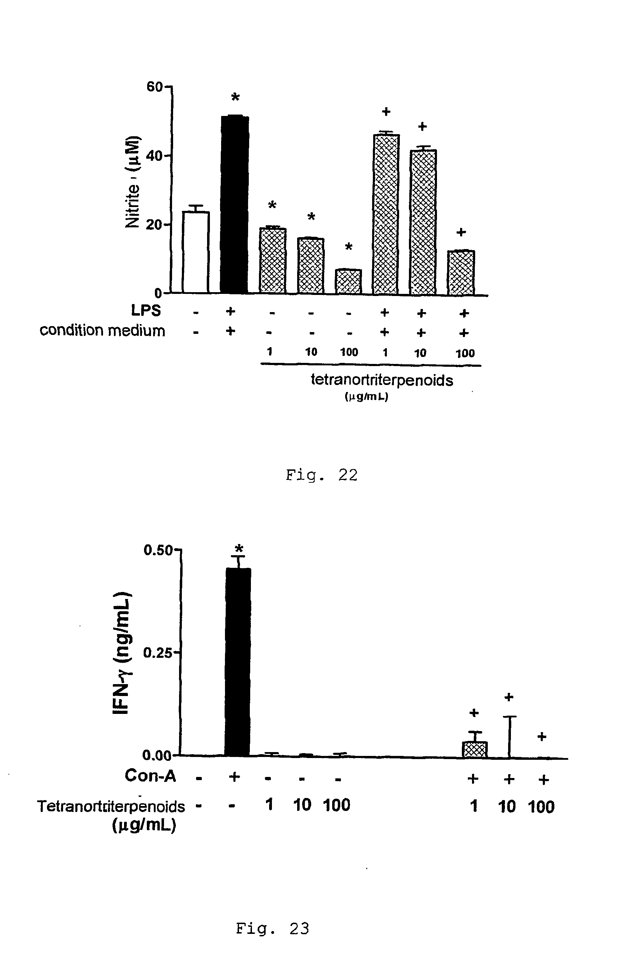

The first experimental model in vitro used for evaluation of anti-inflammatory activity of tetranortriterpenoids was the model of production of nitric oxide by peritoneal macrophages. In figure 22, each column shows the mean of the duplicate of a demonstration experiment, and asterisks and crosses indicate $p \leq 0.05$ when respectively compared to the values of non-stimulated groups (open column) or with the values of group stimulated with LPS in a conditioned environment (closed column).

We observe the production of nitric oxide induced by stimulation for 24 hours with LPS (30mg/mL) and an environment rich in interferon (closed column), basal production of nitric oxide (first open column) and the effect of tetranortriterpenoids (1, 10 and 100 $\mu$g/mL; hachured columns). Basal production (third to fifth columns) of nitric oxide was significantly inhibited with the dose of $\mu$g/mL, and 100$\mu$g/mL was the maximum dosage. In groups stimulated with LPS and with rich interferon environment a discrete inhibition of production of nitric oxide was observed in doses of 1 and 10$\mu$g/mL of tetranortriterpenoids, however the treatment with a dose of 100$\mu$g/mL was able to reduce the production of nitric oxide to values inferior to the basal value.

**b) Evaluation of influence of tetranortriterpenoids isolated from oil of *Carapa guianensis* on the production of interferon-$\gamma$ and TNF-$\alpha$**

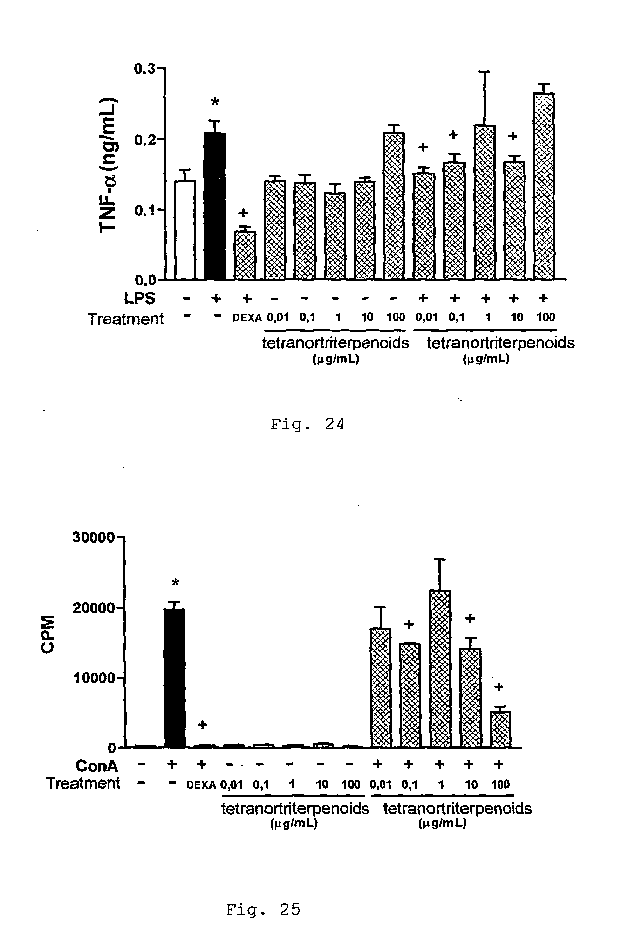

Evaluation of anti-inflammatory and immune modulating activity was made through production of IFN-$\gamma$ by splenocytes and TNF-$\alpha$ by murine peritoneal macrophages. In figures 23 and 24, each column shows the mean of the replica of a demonstration experiment, and asterisks and crosses indicate $p \leq 0.05$ when respectively compared to values of non-stimulated group (open column) or with values of group stimulated (closed column). The production of IFN-y by murine splenocytes was evaluated 24h after the stimulation with Con-A (0.4$\mu$g/well) in the presence or not of crescent doses of tetranortriterpenoids (1, 10, 100$\mu$g/mL), the effect of the tetranortriterpenoids in basal production of IFN-$\gamma$ was also analyzed. In figure 23, we observe that the treatment with the tetranortriterpenoids did not alter the basal production of IFN-$\gamma$ (third to fifth column). The stimulation with Con-A induces production of IFN-$\gamma$ (p<0.05) in 24 hours and the treatment with tetranortriterpenoids significantly inhibited (p<0.05) the production of this cytokine. In the model of production of TNF-$\alpha$ by murine macrophages, we observed that the treatment with tetranortriterpenoids (fourth to seventh columns, figure 24) did not alter the basal production of TNF-$\alpha$ (open column). The glucocorticoid dexamethasone (0.005$\mu$M; third column) inhibited significantly the production TNF (30ng/mL; closed column) by macrophages stimulated with LPS (30ng/mL) for 24h, however, treatment with tetranortriterpenoids was not able to alter basal production of TNF-$\alpha$, but it was able to inhibit (p<0.05), in doses of 0.01, 0.1 and 10$\mu$g/mL, the production of this cytokine induced by LPS.

**c) Evaluation of influence of tetranortriterpenoids isolated from oil of *Carapa guianensis* on the proliferation of lymphocytes**

In figure 25 each column shows the mean of replication of a demonstration experiment, and the asterisks and the crosses indicate $p \leq 0.05$ when respectively compared to the non-stimulated group (open column; first column) or with the group stimulated with Con-A (0.4$\mu$g/well; closed column). We notice the incubation of lymphocytes with the tetranortriterpenoids was not able to alter the basal proliferation of lymphocytes in vitro (fourth to eighth columns) and that the stimulation for 72h with Con-A induces the proliferation of lymphocytes (closed column). Pre-treatment with tetranortriterpenoids inhibited (p<0.05) with doses of 0.1,10 and 100 $\mu$g/mL (columns nine to thirteen) the proliferation of lymphocytes induced by Con-A. The treatment with glucocorticoid dexamethasone (0.005$\mu$M) inhibited the proliferation of lymphocytes significantly (p<0.05).

**d) Evaluation of influence of tetranortriterpenoids isolated from oil of *Carapa guianensis* on the phagocytosis by murine macrophages**

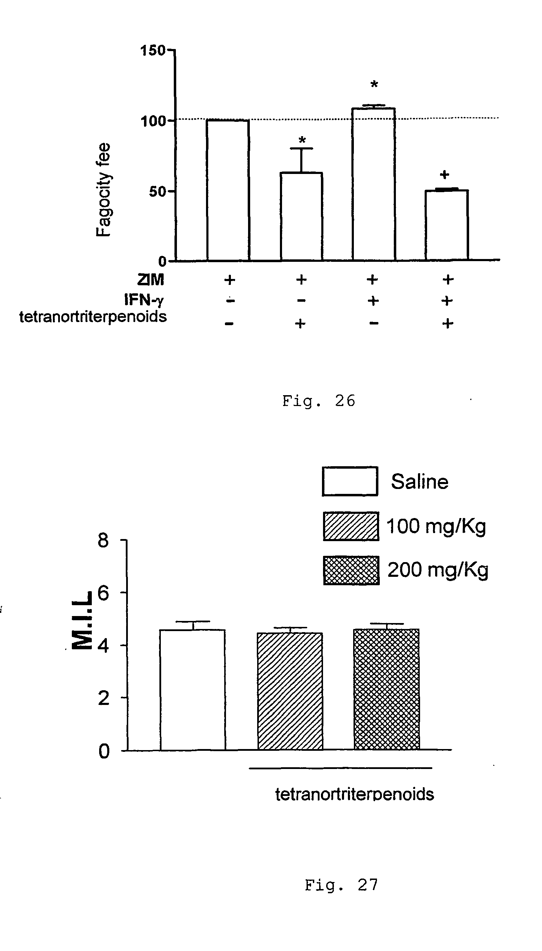

In order to evaluate the modulation of the activity of macrophages we analyzed the effect of tetranortriterpenoids, on the phagocytosis of particles of zimosan by murine macrophages. In figure 26 data was expressed as percentage of the rate of phagocytosis, and each column shows the mean of the triplicate of a demonstration experiment, and the asterisks and crosses indicate $p \leq 0.05$ when respectively compared to the group that received only zimosan ($10^6$ part./mL;

first column) or with the group stimulated with zimosan in the presence of IFN-$\gamma$ (10UI/mL; third column). We observe that the treatment with tetranortriterpenoids (100$\mu$g/mL; second column) significantly inhibited phagocytosis basal of peritoneal macrophages. Treatment of the cells with IFN induced a discrete increase in the rate of phagocytosis and the treatment with tetranortriterpenoids was able to decrease the rate of phagocytosis for values below basal (fourth column; p<0.05).

**Example 22:**

Methodology of essays *in vivo* referring to the induction of acute gastric ulceration by oral administration of tetranortriterpenoids.

Test of acute ulceration induction by tetranortriterpenoids

This essay was performed in animals C57/B110 fasting for 24h with free access to water, maintained in a cage that impeached the ingestion of wood shavings. The animals orally received, 100 or 200 mg/Kg of tetranortriterpenoids in 200 $\mu$L of solution saline with the help of a special curved needle with spherical end. The group was administered only with saline solution, of the same volume. Five hours after the oral administration, mice were sacrificed in a $CO_2$ chamber and were fixed in a rack with adequate pins. The fur was settled with alcohol to avoid interference of furs while withdrawing the stomach. The fur of the mice was suspended with a flat tweezers at the region near the genital organ and an incision was made from this region until the neck area. Then, the skin was forced in the horizontal direction, allowing the abdominal area to be completely visible for manipulation. Through an incision in this region, the stomach was isolated, removed from the abdominal cavity and externally washed with PBS. Each stomach was opened by the smaller curvature, washed and added to polypropylene tubes of 50ml with conic bottom, duly identified and containing PBS. After the removal of all stomachs, they were cautiously placed on a rack and the first two pins were placed in the extremities of the region of the bottom and the other two, in the region of the cavity. With the fixation of all stomachs on the rack, 2-3 drops of PBS were instilled in the gastric mucous of each one of them in order to preserve the humidity and macroscopic analysis of the gastric mucous through a stereoscopic microscope. In the table below, the parameters evaluated are described, considering the following grades of lesion:

**Mild -** when the affected area is < 25%;
**Moderate -** then the area is = 50%;
**Intense -** when the area is > 50%

| Gastric lesions group | Seriousness of lesion | Score | Animal X |
|---|---|---|---|
| 1. Color of mucous | Normal | 1 | |
| | Hyperemic | 2 | |
| | Discolored | 3 | |
| 2. Loss of pleats of mucous | | 1 | |
| 3. Petechia | Mild | 1 | |
| | Moderate | 2 | |
| | Intense | 3 | |
| 4. Edema | Mild | 1 | |
| | Moderate | 2 | |
| | Intense | 3 | |
| 5. Hemorrhage | Mild | 1 | |
| | Moderate | 2 | |
| | Intense | 3 | |
| 6. Loss of mucous | Mild | 1 | |
| | Moderate | 2 | |

(continued)

| Gastric lesions group | Seriousness of lesion | Score | Animal X |
|---|---|---|---|
| | Intense | 3 | |
| 7. Ulcerative lesions | Up to 1mm | 1 | |
| | > 1mm | 1.5xn | |
| | Perforated | 5xn | |
| Total | | | |
| Mean of lesions | | | |
| Pattern deviation | | | |
| Pattern error from the mean | | | |

**Example 23:**

Evaluation of gastric injury *in* vivo after oral administration of tetranortriterpenoids

For the evaluation of ulcerogenic activity of tetranortriterpenoids, doses of 100 and 200 mg/Kg in mice C57/B110 were tested. The analyses were performed 5h after the administration of tetranortritepenoids. Results were expressed as mean of the rate of lesions, as described by Lapa and collaborators (2003) and the pattern error from the mean (E.P.M.), and statistically analyzed through analysis of variance (ANOVA), followed by test of multiple comparisons of Newman-Kewls, or by test T of Student with a level of significance less or equal to 0.05 ($p \leq 0.05$).

Figure 27 shows pre-treatment results of mice with tetranortriterpenoids, with doses of 100 and 200 mg/Kg. Each bar shows the mean $\pm$ E.P.M. of at least 7 animals. The open bar corresponds to the group of animals that received the vehicle (saline). The second bar corresponds to the group that received 100 mg/Kg with tetranortriterpenoids, and the third bar shows the animals that received a dose of 200 mg/Kg. Figure 27 shows that the oral administration of tetranortriterpenoids in mice was not able to induce any alteration in the gastric mucous, according to the evaluation scale described in example 22, in any of the doses tested.

**Claims**

1.   Pharmaceutical composition **characterized by** comprising as active ingredients the components (a) or (b) below:

(a) 5 to 30% of an oil extracted from the seeds of *Carapa guianensis Aublet* and 0.01 % to 5% of tetranortriterpenoids isolated from the oil of Carapa guianensis Aublet, wherein the tetranortriterpenoids are responsible for the pharmaceutical activity;
(b) 0.01% to 5% of tetranortriterpenoids isolated from the oil of Carapa guianensis Aublet, wherein the tetranortriterpenoids are responsible for the pharmaceutical activity;

wherein the composition (a) or (b) further comprising as vehicles and/or additives 0.5 to 6% of emulsion basis; 0.2 to 2% of solid vaseline; 0.05 to 0.1 % of preserving agents; 2 to 20% of humidifying agents; 0.1 to 10% of 1.8 Cineol; and distilled water qsp 100%;
wherein the dosage form of the pharmaceutical composition (a) or (b) is oral or topic.

2.   Pharmaceutical composition according to claim 1 **characterized by** being in liquid, solid or semi-solid form.

3.   Pharmaceutical composition according to claim 1 or 2 **characterized by** the dosage form being a topic formulation selected from the group consisting of cream, ointment, lotion, gel, solution or suspension.

4.   Use of the pharmaceutical composition as defined in any of the claims 1 to 3 in preparation of an anti-allergenic, anti-inflammatory, analgesic and immune modulating medicament.

**Patentansprüche**

1. Pharmazeutische Zusammensetzung, die durch das Enthalten der folgenden Komponenten (a) oder (b) als Wirkstoffe gekennzeichnet ist:

   (a) 5 bis 30 % eines Öls, das aus den Samen von *Carapa guianensis Aublet* extrahiert ist, und 0,01 % bis 5 % an Tetranortriterpenoiden, isoliert aus dem Öl von *Carapa guianensis Aublet,* wobei die Tetranortriterpenoide für die pharmazeutische Aktivität verantwortlich sind;
   (b) 0,01 % bis 5 % an Tetranortriterpenoiden, isoliert aus dem Öl von *Carapa guianensis Aublet,* wobei die Tetranortriterpenoide für die pharmazeutische Aktivität verantwortlich sind;

   wobei die Zusammensetzung (a) oder (b) ferner als Formulierungshilfsmittel und/oder als Zusatzstoffe 0,5 bis 6 % Emulsionsgrundlage; 0,2 bis 2 % feste Vaseline; 0,05 bis 0,1 % Konservierungsmittel; 2 bis 20 % an Befeuchtungsmitteln; 0,1 bis 10 % 1.8-Cineol und destilliertes Wasser qsp auf 100 % enthält;
   wobei die Dosierungsform der pharmazeutischen Zusammensetzung (a) oder (b) oral oder topisch ist.

2. Pharmazeutische Zusammensetzung nach Anspruch 1, **dadurch gekennzeichnet, dass** sie in einer flüssigen, festen oder halbfesten Form ist.

3. Pharmazeutische Zusammensetzung nach Anspruch 1 oder 2, **dadurch gekennzeichnet, dass** die Dosierungsform ein topische Formulierung ist, die ausgewählt ist aus der Gruppe, die aus Creme, Salbe, Lotion, Gel, Lösung oder Suspension besteht.

4. Verwendung der pharmazeutischen Zusammensetzung nach einem der Ansprüche 1 bis 3 in der Zubereitung von einem anti-allergenen, entzündungshemmenden, schmerzlindernden und immunmodulatorischen Medikament.

**Revendications**

1. Composition pharmaceutique **caractérisée en ce qu'**elle comprend comme ingrédients actifs les composants (a) ou (b) ci-dessous :

   (a) 5 % à 30 % d'une huile extraite des graines de *Carapa guianensis Aublet* et 0,01 % à 5 % de tétranortriterpénoïdes isolés de l'huile de *Carapa guianensis Aublet,* dans laquelle les tétranortriterpénoïdes sont responsables de l'activité pharmaceutique ;
   (b) 0,01 % à 5 % de tétranortriterpénoïdes isolés de l'huile de *Carapa guianensis Aublet,* dans laquelle les tétranortriterpénoïdes sont responsables de l'activité pharmaceutique ;

   dans laquelle la composition (a) ou (b) comprend en outre des supports et/ou des adjuvants dans une quantité de 0,5 à 6 % de base d'émulsion ; 0,2 à 2 % de vaseline solide ; 0,05 à 0,1 % d'agents conservateurs ; 2 à 20 % d'agents d'humidification ; 0,1 à 10 % de 1,8-Cinéole ; et de l'eau distillée qsp 100 % ;
   dans laquelle la forme de dosage de la composition pharmaceutique (a) ou (b) est orale ou topique.

2. Composition pharmaceutique selon la revendication 1, **caractérisée en ce qu'**elle est sous une forme liquide, solide, ou semi-solide.

3. Composition pharmaceutique selon la revendication 1 ou la revendication 2, **caractérisée en ce que** la forme de dosage est une formulation topique sélectionnée parmi le groupe constitué de crème, onguent, lotion, gel, solution ou suspension.

4. Utilisation de la composition pharmaceutique telle que définie dans l'une quelconque des revendications 1 à 3, en préparation d'un médicament anti-allergique, anti-inflammatoire, analgésique et modulateur immunitaire.

Fig. 1

Fig. 2

Fig. 3

Fig. 4

Fig. 5

Fig. 6

Fig. 7A

Fig. 7B

Fig. 8A

Fig. 8B

Fig. 9

Fig. 10

Fig. 11A

Fig. 11B

Fig. 12

Fig. 13

Fig. 14

Fig. 15A

Fig. 15B

Fig. 15C

Fig. 16

Fig. 17

Fig. 18

Fig. 19

35

Fig. 20

Fig. 21

Fig. 22

Fig. 23

Fig. 24

Fig. 25

Fig. 26

Fig. 27

**EP 1 819 352 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- BR PI0108940
- US 6730332 B
- JP 2001151634 A
- JP 2002020243 A
- BR PI9301949
- BR PI9302004
- BR PI9302006
- US 5958421 A
- US 4603137 A